# EUROPEAN PATENT APPLICATION

(11) **EP 1 057 484 A1**
(43) Date of publication of application: **06.12.2000**
(21) Application number: 99905260.8
(22) Date of filing: 22.02.1999
(51) Int. Cl.: A61K 31/30, C07D 403/04

(54) **CELL DEATH INHIBITORS**

(30) Priority: 23.02.1998 JP 4014798; 23.02.1998 JP 4014898; 10.06.1998 JP 16211898; 10.06.1998 JP 16211998
(71) Applicant: SAGAMI CHEMICAL RESEARCH CENTER, Sagamihara-shi, Kanagawa 229-0012 (JP); Asakai, Rei, Ohmiya-shi, Saitama 330-0801 (JP)
(72) Inventor: ASAKAI, Rei, 403-go, Kitaohmiyajutaku 1-gotoh, Ohmiya-shi, Saitama 330-0801 (JP); SODEOKA, Mikiko, Sendai-shi Miyagi 980-8577 (JP); FUJITA, Mikako, Tokushima-shi Tokushima 770-0045 (JP); KATOH, Miho, Ebina-shi, Kanagawa 243-0414 (JP)
(74) Representative: Albrecht, Thomas, Dr.
(86) International application number: JP9900772
(87) International publication number: WO9942100

(57) **Abstract**

The present invention provides cell death inhibitors comprising, as an active ingredient, an indole derivative represented by the following formula (I) which is expected as a preventive or a remedy for the progress of various diseases wherein cell death participates in progress and exacerbation thereof, drugs, preservatives for organs, tissues and cells, and assay systems using primary cultured cells:

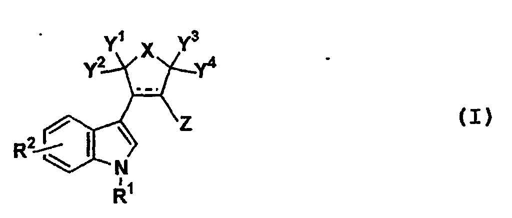

## Description

### Technical Field

The present invention relates to a cell death inhibitor capable off inhibiting cell death induced by various substances in living body or foreign stimulants, or stimuli such as temperature, radiation and so on; its use as drugs for treating neurodegenerative diseases, diseases of circulatory organs, hepatitis, renal diseases, inflammatory skin disorders, radiation disorders, viral diseases, prion diseases, functional deficiency of transplanted organs, or the like, or preventing progress of the symptoms of the diseases; use as preservatives for organs, tissues and cells isolated from a living body; and an assay method for searching a cell death inhibitor.

### Background Art

Progress of the study as to cell death have revealed that cell death of cells essential for living body, particularly apoptosis is involved in progress and exacerbation of a variety of diseases (*Science*, Vol. 267, p. 1456, 1995). Apoptosis is a type of cell death in which cells commit a suicide using their own molecular machinery, characterized generally by (1) chromatin aggregation, (2) cell shrinkage, (3) blebbing of plasma membrane (formation of processes), (4) nuclear fragmentation, (5) formation of apoptotic bodies, (6) DNA fragmentation, and (7) phagocytosis (scavenging cell debris) by neighboring cells and macrophages. In contrast, there is another type of cell death, called necrosis, characterized by cell swelling and lysis, which occurs without executing the apoptotic processes when cells are exposed to excessive radiation, heat, noxious stimulants or the like. However, the cell death caused by the own molecular machinery does not always show a full set of the apoptosis characteristics described above, depending on species of cells, environments under which cells are present, and species and strength of cell death stimulants. Likewise, necrosis in view of pathology sometimes contains a cell death which some own molecular machinery is responsible for. In the present invention, such cell death is also included in apoptosis.

Examples of the diseases whose progress and exacerbation are caused by apoptotic cell death are as follows: neurodegenerative diseases such as Alzheimer's disease (*Bio Science terminology library*: apoptosis/separate volume of *Jikken Igaku (Experimental Medicine)*, p. 168, 1996), spinal muscular atrophy (SMA) (*Bio Science terminology library*: apoptosis/separate volume of *Jikken Igaku (Experimental Medicine)*, p. 173, 1996), amyotrophic lateral screrosis (ALS) (*Bio Science terminology library*: apoptosis/separate volume of *Jikken Igaku (Experimental Medicine*), p. 176, 1996), Parkinson's disease (*J. Neurochem.*, Vol. 69, p. 1612, 1997), Huntington's disease (*J. Neurosci*., Vol. 15, p. 3775, 1995), pigmentary degeneration of the retina and glaucoma (*Bio Science terminology library*: apoptosis/separate volume of *Jikken Igaku (Experimental Medicine*), p. 196, 1996), cerebellar degeneration and neonatal jaundice (*Progress in Drug Research*, Vol. 48, p. 55, 1997); myasthenia gravis (*J. Clinical Investigation*, Vol. 99, p. 2745, 1997); brain ischemia from apoplexy and the like, and successive delayed neuronal death (DND) (*Bio Science terminology library*: apoptosis/separate volume of *Jikken Igaku (Experimental Medicine*), p. 180, p. 182, 1996), ischemic heart disease due to myocardial infarction (myocardial ischemia and disorder after reperfusion), viral myocarditis, autoimmune myocarditis (congestive cardiomyopathy and chronic myocarditis), myocardial disorders or death due to hypertrophic heart and heart failure, arrythmogenic right ventricular cardiomyopathy (*Bio Science terminology library*: apoptosis/separate volume of *Jikken Igaku (Experimental Medicine*), p. 198, 1996; *Kekkan to Naihi (Blood Vessel and Endothelium)*, Vol. 7, p. 357, p. 364, p. 370, 1997); alcoholic hepatitis, viral hepatitis (*Bio Science terminology library*: apoptosis/separate volume of *Jikken Igaku (Experimental Medicine)*, p. 190, 1996), renal diseases such as glomerulonephritis, hemolytic uremic syndrome and the like (*Bio Science terminology library*: apoptosis/separate volume of *Jikken Igaku (Experimental Medicine)*, p. 192, 1996), acquired immunodeficiency syndrome (AIDS) (*Bio Science terminology library*: apoptosis/separate volume of *Jikken Igaku (Experimental Medicine*), P. 156, 1996; *Ketsueki, Meneki*, *Shuyou (Blood, Immunity, Cancer*), Vol.2, P. 432, 1997), inflammatory skin disorders such as toxic epidermal necrolysis (TEN) and multiform exudative erythema, alopecia, graft versus host disease (GVH) (*Bio Science terminology library*: apoptosis/separate volume of *Jikken Igaku (Experimental Medicine)*, P. 194, 1996), radiation disorders (*Bio Science terminology library*: apoptosis/separate volume of *Jikken Igaku (Experimental Medicine*), p. 160, 1996), side effects due to anti-cancer drugs, anti-viral drugs and the like, disorders due to toxic agents such as sodium azide, potassium cyanide and the like (*Bio Science terminology library*: apoptosis/separate volume of *Jikken Igaku (Experimental Medicine)*, p. 162, 1996), sepsis (*Critical Care Medicine*, Vol. 25, p. 1298, 1996), osteomyelo-dysplasia such as aplastic anemia and the like (*Leukemia*, Vol. 7, P. 144, 1993), insulin dependent diabetes (*Diabetes*, Vol. 44, p. 733, 1995), prion diseases such as Creutzfeldt-Jakob's disease (*J. Neural Transmission*, Supplementum, Vol. 50, p. 191, 1997), and so on. In organ transplantation, it has been suggested that apoptosis due to active oxygen species and various chemical mediators generated after reperfusion of anoxic organs by isolation or cardiac arrest of a donor is responsible for functional deficiency of transplanted organs (for example, *Ishoku (Transplantation)*, Vol. 27, p. 15, 1992). Probably, rejection reaction after transplantation of an organ, tissues, or cells may be a result of apoptosis of the transplanted cells, which occurs when they are attacked by recipient immune cells. It is thus reasonably concluded that chemical compounds capable of inhibiting cell death can be a promising drug that heals these diseases effectively, or inhibits or stops progress and exacerbation of the symptoms of these diseases.

In the transplantation of organs or tissues, a graft survival rate after transplantation depends on the preserving conditions of the organs or tissues isolated from a donor. Accordingly, it is expected to improve organ and tissue preservation by adding chemical compounds inhibiting cell death into preservation liquids for the organs and tissues. Unlike immortalized cells or cancer cells, primary cultured cells isolated from a living body are usually difficult to culture *in vitro*. For long time culture, additives including various growth factors are required in the culture medium at an appropriate concentration depending on species of the cells, and apoptosis easily occurs when the culture conditions are improper. When cells are cultured for research or medical purposes, it is expected that addition of a chemical compound inhibiting cell death would lead successful cell culture.

Apoptosis is known to be triggered by a wide variety of physiological substances such as cytokines including interleukins, hormones including glucocorticoids, excitotoxic amino acids including glutamic acid and NMDA, and membrane proteins represented by Fas ligand, depending on cell types. It is also triggered by deprivation of a specific growth factor or the like in some cell types. There are common apoptosis triggers irrespective of cell type, such as active oxygen species generators including hydrogen peroxide and the like, NO generators including SNP and the like, heat, and radiation. A number of chemical compounds are also reported to be able to induce apoptosis. Recent studies have shown that apoptotic signal transduction systems where a variety of signal transduction systems participate at the upstream, appear to converge on caspase activating mechanisms at the downstream, the caspases being a series of cysteine protease (*Cell*, Vol. 91, p. 443, 1997), though their precise molecular mechanisms should be investigated in future.

Substances heretofore known as apoptosis inhibitors are, depending on species of the cells, a variety of growth factors and nutrient factors, physiological inhibitors such as hormones and the like, antioxidants such as N-acetylcysteine and the like, and modified peptide-type caspase inhibitors. Among these, some of peptide-type growth factors and neurotropic factors have been clinically used for the recovery of hematopoietic cells depleted after chemotherapy and for preventing cell death of neurons from neuro-degenerative diseases and trauma (*Proc. Natl. Acad. Sci*. *U.S.A.*, Vol. 90, p. 7951, 1993; *Nature*, Vol. 367, p. 368, 1994; *Proc. Natl. Acad. Sci. U.S.A*., Vol. 89, p. 11249, 1992). The antioxidants and caspase inhibitors are only used in experiments of the cell level. Thus, it has been desired to develop a non-peptide type low molecular weight apoptosis inhibitor which is more stable *in vivo* and can be orally administered. Furthermore, since it is rare case that all apoptosis-triggering physiological factors and its inhibiting factors of the individual cells have been successfully identified in actual diseases, there is a demand for an entirely new type of cell death inhibitor, especially an apoptosis inhibitor which is also expected to be beneficial for the diseases where the factors are unidentified.

On the other hand, 2-halo-3-indolylmaleimide derivatives have been reported as synthetic intermediates for bisindolylmaleimide derivatives and indolocarbazole derivatives which have a protein kinase C inhibiting activity (for example, WO 95/30682, WO 97/34890, WO 97/09339, Japanese Patent Laid-Open No. 174778/1990). However, there are no reports on their function of inhibiting cell death.

In addition, bisindolylmaleimide derivatives have been reported that they can inhibit protein kinases, especially protein kinase C (for example, Japanese Patent Laid-Open No. 306974/1990, DE 3835842), and their function as an anti-cancer drug is known. But, there is no report on their function of inhibiting cell death.

At present, Euro-Collins'solution and University of Wisconsin solution are generally used as organ preservation solutions for transplantation (*IShoku (Transplantation)*, Vol. 27, p. 172, 1992). Supplementation of antioxidants and radical scavengers to such preservation solutions in order to ameliorate damages of active oxygen has been reported to have beneficial effects on organ preservation (for example, *IShoku (Transplantation)*, Vol. 27, p. 15, 1992; Vol. 26, p. 62, 1991; Vol. 25, p. 596, 1990; *Trans Proc*., Vol. 17, p. 1454, 1985). However, the organ preservation is not fully sufficient, and higher graft survival rate is still desired.

As assay methods of cell death inhibitors, screening is widely carried out using immortalized cells and cancer cells which are easy to culture. However, most of these types of cells are inherently abnormal in the apoptotic mechanisms, and thus immortalized. Therefore, there is a possibility that compounds effective in inhibiting death of normal cells would exhibit no activity on the cells and a more convenient assay method has been sought.

### Disclosure of the Invention

An object of the present invention is to provide a drug useful for inhibiting death of cells, the drug being expected as a preventive or a remedy for the progress of various diseases wherein cell death participates in progress and exacerbation thereof.

As a result of the extensive studies for achieving the above, the present inventors have found that the below-described derivatives exhibit a cell death inhibiting action and have accomplished the invention.

Namely, the present invention provides a cell death inhibitor comprising, as an active ingredient, an indole derivative represented by the following formula (I): 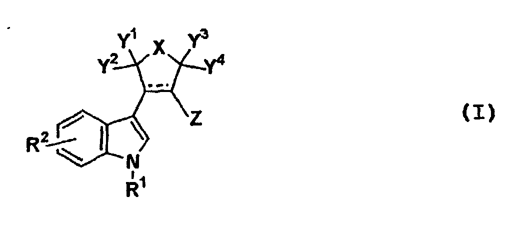 wherein X represents an oxygen atom or N-R⁵; Z represents a hologen atom or 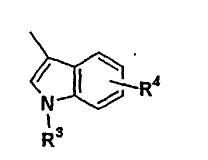 R¹ and R³ each independently represents a hydrogen atom, an alkyl group which may possess substituent(s), an alkenyl group which may possess substituent(s), an alkynyl group which may possess substituent(s), an aryl group which may possess substituent(s), an acyl group which may possess substituent(s), an alkoxy- or aryloxycarbonyl group which may possess substituent(s), an alkyl- or arylthiocarbonyl group which may possess substituent(s), an aminocarbonyl group which may possess substituent(s), an alkyl- or arylsulfonyl group which may possess substituent(s), an alkoxyl group or an aryloxy group which may possess substituent(s), or a hydroxyl group; R² and R⁴ each represents substituent(s) on an indole ring, in which number and position (2-, 4-, 5-, 6-, or 7-position as position number of the indole ring) of the substituent(s) and kinds of the substituent(s) may be the same or different, and represents a hydrogen atom, an alkyl group which may possess substituent(s), an alkenyl group which may possess substituent(s), an alkynyl group which may possess substituent(s), an aryl group which may possess substituent(s), an acyl group which may possess substituent(s), an alkoxy- or aryloxycarbonyl group which may possess substituent(s), an alkyl- or arylthiocarbonyl group which may possess substituent(s), an aminocarbonyl group which may possess substituent(s), an alkyl- or arylsulfonyl group which may possess substituent(s), an alkoxyl group or an aryloxy group which may possess substituent(s), an alkyl-or arylthio group which may possess substituent(s), a hydroxyl group, a carboxyl group, a cyano group, a nitro group, an amino group which may possess substituent(s), or a halogen atom; R⁵ represents an alkyl group which may possess substituent(s), an alkenyl group which may possess substituent(s), an alkynyl group which may possess substituent(s), an aryl group which may possess substituent(s), an alkoxyl group or an aryloxy group which may possess substituent(s), an amino group which may possess substituent(s), a hydroxyl group, or a hydrogen atom; Y¹ and Y², and Y³ and Y⁴ each independently represent two hydrogen atoms or a hydrogen atom and a hydroxyl group, or are combined to form a carbonyl group; and R¹ and R², R¹ and R³, R³ and R⁴, or R² and R⁴ may be combined to form a hydrocarbon chain or a hydrocarbon chain containing hetero atom(s) which may possess substituent(s); and in the formula, the bond accompanying a dotted line represents a double bond or a single bond, or a pharmaceutically acceptable salt thereof; a drug for treating or preventing progress of symptoms, through inhibiting death of neurons, of neurodegenerative diseases such as Alzheimer's disease, spinal muscular atrophy (SMA), amyotrophic lateral screrosis (ALS), Parkinson's disease, Huntington's disease, pigmentary degeneration of the retina, glaucoma, or cerebellar degeneration; a drug for treating or preventing progress of symptoms, through inhibiting death of neurons, of neonatal jaundice; a drug for treating or preventing progress of symptoms, through inhibiting death of cells, of myasthenia gravis; a drug for treating or preventing progress of symptoms, through inhibiting death of neurons, of brain ischemia from apoplexy and the like, and successive delayed neuronal death (DND); a drug for treating or preventing progress of symptoms, through inhibiting death of myocardial cells, of ischemic heart disease due to myocardial infarction (myocardial ischemia and disorder after reperfusion), viral myocarditis, autoimmune myocarditis, myocardial disorders or death due to hypertrophic heart and heart failure, or arrythmogenic right ventricular cardiomyopathy; a drug for treating or preventing progress of symptoms, through inhibiting death of hepatic cells, of alcoholic hepatitis or viral hepatitis; a drug for treating or preventing progress of symptoms, through inhibiting death of renal cells, of renal diseases such as glomerulonephritis, hemolytic uremic syndrome and the like; a drug for treating or preventing progress of symptoms, through inhibiting excessive death of T-cells, of acquired immunodeficiency syndrome (AIDS); a drug for treating or preventing progress of symptoms, through inhibiting cell death, of inflammatory skin disorders such as toxic epidermal necrolysis (TEN), multiform exudative erythema and the like, alopecia, or graft versus host disease (GVH); a drug for treating or preventing disorders or side effects, through inhibiting cell death, of radiation disorders or disorders due to toxic agents including side effects due to drugs such as anti-cancer drugs, anti-viral drugs and the like; a drug for treating or preventing progress of symptoms, through inhibiting cell death, of sepsis; a drug for treating or preventing progress of symptoms, through inhibiting death of cells derived from bone marrow, of osteomyelo-dysplasia such as aplastic anemia and the like; a drug for treating or preventing progress of symptoms, through inhibiting cell death, of insulin dependent diabetes; a drug for treating or preventing progress of symptoms, through inhibiting death of neurons, of prion diseases; a drug for treating or preventing functional deficiency of transplanted organs, tissues or cells at transplantation of organs, tissues or cells; a preservative for organs, tissues and cells; and an assay method for cell death inhibiting substances, comprising applying a cell death-inducing stimulus to primary cultured cells in the presence of a test compound or adding a test compound just after applying a cell death-inducing stimulus, followed by evaluating a ratio of cell death.

Furthermore, the present invention provides a medicament comprising, as an active ingredient, a 2-halo-3-indolylmaleimide derivative represented by the following formula (II): 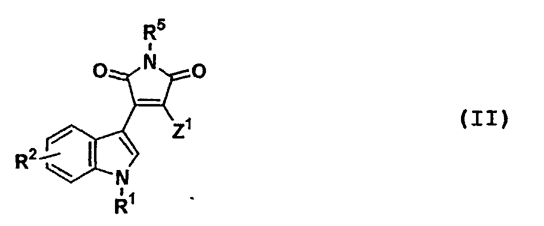 wherein Z¹ represents a halogen atom; and R¹, R² and R⁵ have the same meanings as described above, or a pharmaceutically acceptable salt thereof.

The following will explain the present invention in detail.

### Best Mode for Carrying Out the Invention

The indole derivatives according to the present invention can be synthesized following to known methods, for example, *Tetrahedron*, Vol. 44, p. 2887, 1988; *J. Biol Chem*., Vol. 266, p. 15771, 1991; *J. Med. Chem*., Vol. 35, p. 177, p. 994, 1992; *J. Med. Chem*., Vol. 36, p. 21, 1993, or analogous methods thereof, other than the methods shown in Referential Examples, or are commercially available (from CALBIOCHEM; LC Laboratory, etc.).

In the present description, the alkyl group in the "alkyl group which may possess substituent(s)" may be any of linear, branched, or cyclic, and examples include alkyl groups having 1 to 30 carbon atoms, such as a methyl group, an ethyl group, a propyl group, an isopropyl group, a butyl group, an isobutyl group, an s-butyl group, a t-butyl group, a pentyl group, an isopentyl group, a neopentyl group, a heryl group, a heptyl group, an octyl group, a nonyl group, a decyl group, a cyclopropyl group, a cyclobutyl group, a cyclopentyl group, a cyclohexyl group, a cycloheptyl group, an undecyl group, a dodecyl group, a tridecyl group, a tetradecyl group, a pentadecyl group, a hexadecyl group, a 14-methylpentadecyl group, a 6-methylpentadecyl group, an octadecyl group, an eicosyl group, a tetracosyl group, and the like.

In the present description, the alkenyl group in the "alkenyl group which may possess substituent(s)" may be any of linear, branched, or cyclic, and examples include alkenyl groups having 2 to 30 carbon atoms, such as an allyl group, a vinyl group, a crotyl group, a 1-penten-1-yl group, a 2-penten-1-yl group, a 3-penten-1-yl group, a 1-hexen-1-yl group, a 2-hexen-1-yl group, a 3-hexen-1-yl group, a 2-cyclohexenyl group, a 2-cyclopentenyl group, a 8-heptadecen-1-yl group, a 8,11-heptadecadien-1-yl group, a 8,11,14-heptadecatrien-1-yl group, a 4,7,10,13-nonadecatetraen-1-yl group, a 9-octadecen-1-yl group, a 9,12-octadecadien-1-yl group, a 9,12,15-octadecatrien-1-yl group, a 6,9,12-octadecatrien-1-yl group, a 5,8,11,14-eicosatetraen-1-yl group, a 5,8,11,14,17-eicosapentaen-1-yl group, a 4,7,10,13,16,19-docosahexane-1-yl group, and the like.

In the present description, the alkynyl group in the "alkynyl group which may possess substituent(s)" may be any of linear, branched, or cyclic one, and examples include alkynyl groups having 2 to 30 carbon atoms, such as an ethynyl group, a propargyl group, a 1-pentyn-1-yl group, a 2-pentyn-1-yl group, a 3-pentyn-1-yl group, a 1-octyn-1-yl group, a 8-heptadecyn-1-yl group, and the like

In the present description, the aryl group in the "aryl group which may posses substituent(s)" includes a heteroaryl group, and examples include a phenyl group, a naphthyl group, an anthranyl group, a pyrenyl group, a biphenyl group, a 4-pyridyl group, a 2-pyridyl group, a pyrimidinyl group, a pyrazinyl group, a piperazinyl group, a pyrazolyl group, an imidazolyl group, a quinolyl group, a pyrrolyl group, an indolyl group, a furyl group, and the like.

In the present description, the acyl group in the "acyl group which may possess substituent(s)" may be any of linear, branched, cyclic, saturated, unsaturated, aliphatic or aromatic, and examples include acyl groups having 2 to 30 carbon atoms, such as an acetyl group, a propionyl group, an isopropionyl group, a pivaloyl group, an oleoyl group, a cyclohexylcarbonyl group, an acryloyl group, a crotonoyl group, a benzoyl group, a naphthoyl group, a nicotinoyl group, and the like.

In the present description, the alkoxy- or aryloxycarbonyl group in the "alkoxy- or arylorycarbonyl which may possess substituent(s)" may be any of linear, branched, cyclic, saturated, unsaturated, aliphatic or aromatic, and examples include a methoxycarbonyl group, an ethoxycarbonyl group, a propyloxycarbonyl group, an isopropyloxycarbonyl group, a butoxycarbonyl group, an s-butoxycarbonyl group, a t-butoxycarbonyl group, a cyclopentyloxycarbonyl group, a cyclohexyloxycarbonyl group, a benzyloxycarbonyl group, an allyloxycarbonyl group, a phenyloxycarbonyl group, a pyridyloxycarbonyl group, and the like.

In the present description, the alkyl- or arylthiocarbonyl group in the "alkyl- or arylthiocarbonyl which may possess substituent(s)" may be any of linear, branched, cyclic, saturated, unsaturated, aliphatic or aromatic, and examples include a methylthiocarbonyl group, an ethylthiocarbonyl group, a propylthiocarbonyl group, an isopropylthiocarbonyl group, a butylthiocarbonyl group, a t-butylthiocarbonyl group, a cyclopentylthiocarbonyl group, a cyclohexylthiocarbonyl group, a benzylthiocarbonyl group, a phenylthiocarbonyl group, a pyridylthiocarbonyl group, and the like.

In the present description, the "aminocarbonyl which may possess substituent(s)" may be an unsubstituted carbamoyl group, or a carbamoyl which is substituted by alkyl group(s) which may possess substituent(s), aromatic group(s) which may possess substituent(s), a hydroxyl group, alkoxyl group(s) which may possess substituent(s), amino group(s) which may possess substituent(s), and the like, and examples include a carbamoyl group, an ethylaminocarbonyl group, a propylaminocarbonyl group, an isopropylaminocarbonyl group, a butylaminocarbonyl group, a t-butylaminocarbonyl group, a cyclopentylaminocarbonyl group, a cyclohexylaminocarbonyl group, a benzylaminocarbonyl group, a phenylaminocarbonyl group, a pyridylaminocarbonyl group, and the like.

In the present description, the alkyl- or arylsulfonyl group in the "alkyl or arylsulfonyl which may possess substituent(s)" may be any of linear, branched, cyclic, saturated, unsaturated, aliphatic or aromatic, and examples include a methanesulfonyl group, an ethanesulfonyl group, a benzenesulfonyl group, a cyclohezanesulfonyl group, a naphthalenesulfonyl group, and the like.

In the present description, the alkoxyl group or aryloxy group in the "alkoxyl group or an aryloxy which may possess substituent(s)" may be any of linear, branched, cyclic, saturated, unsaturated, aliphatic or aromatic, and examples include alkoxy groups or aryloxy groups having 2 to 30 carbon atoms, such as a methoxy group, an ethoxy group, a propyloxy group, a t-butoxy group, an allyloxy group, a cyclopentyloxy group, a cyclohexyloxy group, a benzyloxy group, a phenoxy group, and the like.

In the present description, the alkyl- or arylthio group in the "alkyl- or arylthio which may possess substituent(s)" may be any of linear, branched, cyclic, saturated, unsaturated, aliphatic or aromatic, and examples include alkyl- or arylthio groups having 2 to 30 carbon atoms, such as a methylthio group, an ethylthio group, a propylthio group, a t-butylthio group, an allylthio group, a cyclopentylthio group, a cyclohexylthio group, a benzylthio group, a phenylthio group, and the like.

In the present description, the "amino group which may possess substituent(s)" may be an unsubstituted amino group, or an amino which is substituted by alkyl group(*s*), aromatic group(s), and the like, and examples include an ethylamino group, a propylamino group, an isopropylamino group, a butylamino group, a t-butylamino group, a benzylamino group, a phenylamino group, a pyridylamino group, a piperazinyl group, an indolinyl group, and the like.

In the present description, the "halogen atom" includes a fluorine atom, a chlorine atom, a bromine atom, and an iodine atom.

The examples of substituents which may be present in the above-described alkyl group, alkenyl group, alkynyl group, aryl group, acyl group, alkoxycarbonyl group, aryloxycarbonyl group, alkylthiocarbonyl group, arylthiocarbonyl group, aminocarbonyl group, alkoxyl group, aryloxy group, alkylthio group, arylthio group, amino group, and the like include alkyl groups, alkenyl groups, alkynyl groups, aryl groups, acyl groups, alkoxycarbonyl groups, aryloxycarbonyl groups, alkylthiocarbonyl groups, arylthiocarbonyl groups, aminocarbonyl groups, alkoxyl groups, aryloxy groups, alkylthio groups, arylthio groups, and particular examples thereof are the same as described above. The other substituents may be exemplified by an amino group, halogen groups, a nitro group, amino groups (which may possess substituent(s) such as acyl group(s), alkoxycarbonyl group(s), aryloxycarbonyl group (s), carbamoyl group (s), substituted sulfonyl group(s), alkyl group(s), cycloalkyl group(s), aryl group(s), and the like), a cyano group, a hydroxyl group, a carboxyl group, and the like, as well as aralkyl groups such as a benzyl group, a phenethyl group, a naphthylmethyl group, and the like.

As to the pharmaceutically acceptable salts, the compound having an acid part may formed a salt with an inorganic base or organic base, for example, an alkaline metal salt such as a sodium salt, a potassium salt, or the like; an alkaline earth metal salt such as a calcium salt, a magnesium salt, or the like; an ammonium salt; an aliphatic or heteroaromatic amine salt such as a triethylamine salt, an ethanolamine salt, a lysine salt, an arginine salt, a quinoline salt, or the like; a quaternary ammonium salt such as tetramethylammonium or the like. The compound having a basic part may form a salt with an inorganic or organic acid, for example, hydrochloride, bromate, iodate, sulfate, nitrate, phosphate, citrate, tartrate, malate, lactate, salicylate, malonate, fumarate, succiniate, oxalate, ascorbate, or the like.

The compound according to the present invention may be applied as medicament in any form selected from various forms, for example, formulations for oral administration such as tablets, capsules, powders, granules, or liquids; and formulations for parenteral administration such as injections, rectal suppositories, formulations for external use on skin, inhalant, and the like.

Solid formulations can be prepared in a form of tablets, capsules, granules, or powders by themselves, or can be prepared with using suitable additive(s). Examples of such additives include sugars such as lactose or glucose; starches; fatty acids such as stearic acid; inorganic salts such as magnesium metasilicate aluminate or anhydrous calcium phosphate; synthetic polymers such as polyvinylpyrrolidone or polyalkylene glycol; fatty acid salts such as calcium stearate or magnesium stearate; alcohols such as stearyl alcohol or benzyl alcohol; synthetic cellulose derivatives such as methyl cellulose, ethyl cellulose, carboxymethyl cellulose, or hydroxypropyl cellulose; other conventional additives such as water, gelatin, talc, vegetable oils, acacia, or the like.

Liquid formulations are prepared in a form of suspensions, syrups, or injections by using suitable additive(s) conventionally used in liquid formulations such as water, alcohols, vegetable-derived oils including soybean oil, peanut oil, sesame oil, etc.

Especially, examples of suitable solvents for injections include distilled water for injection, aqueous lidocain hydrochloride solution, physiological saline, aqueous glucose solution, ethanol, liquids for intravenous injection such as aqueous solutions of citric acid and sodium citrate, electrolyte solution, and the like, or mixtures Thereof. These injections may be a form of pre-dissolved one, and also a form for dissolving before use, which is composed of powder itself or powder with suitable additive(s).

The rectal suppositories may be prepared either by melting an active ingredient and base material(s) such as cacao butter, tri-, di- and monoglyceride of a fatty acid, polyethylene glycol, and the like under heating; charging the melt into a mold; and then cooling it; or by dissolving an active ingredient into polyethylene glycol, soybean oil, or the like and then covering it with gelatin film.

In the preparation of formulations for external use on skin, an active ingredient is added to vaseline, bees wax, liquid paraffin, polyethylene glycol, etc., followed by either kneading it, if necessary under heating, to form ointments, or kneading it with an adhesive such as rosin, a polymer of alkyl acrylate, etc. and spreading the kneaded one on unwoven cloth such as polyethylene or the like to form tapes.

In the preparation of inhalant, an active ingredient is dissolved or dispersed into a propellant such as fron gas, and then a pressure container is filled up to form aerosols.

Preferred dosage of the compound according to the present invention varies depending on kinds of the compositions blended, dosing times and diseases to be treated, and also ages, body weights, and symptoms of patients, but may be ordinarily in an amount of about 1 to 1000 mg a day, preferably 5 to 500 mg, and they may be administered in one or several dosage units per day.

The organs relating to the present invention may be all organs, such as heart, lung, liver, kidney, pancreas, and intestine.

The tissues relating to the present invention may be all tissues, such as skin, cornea, bone marrow, vascular systems, and bone.

In the present invention, cells expected to have effects on maintenance of the cell function by transplantation or the preservation may be all types of cells (normal various cells, immortalized cell lines, cancer cells, and cells that are modified by genetic recombination techniques for disease treatment and research purposes), such as vascular cells, islet cells of Langerhans, epidermal cells, neuronal cell, and germ stem cells.

As far as an administration method is concerned, when the chemical compounds according to the present invention are used for the preservation of organs, tissues, and cells, various routes can be selected. For example, the present compound or a pharmaceutically acceptable salt thereof can be added to a culture medium or preservation solution containing appropriate salts and nutrients. In case of organ transplantation, it can be also administered intravenously or at perfusion to a donor prior to the organ isolation.

As far as the primary cultured cells used for assay method of the present invention are concerned, all types of cells capable of isolating from animals and culturing are included, for example, neurons, hepatic cells, kidney cell, skin cells, heart muscle cells, vascular endothelial cells, blood cells such as white blood cells, red blood cells, etc., ovarian cells, and so on. Particularly, ovarian granulosa cells are preferable since their highly synchronized cell death in response to various apoptotic stimuli is easily observed under a microscopy.

As far as stimuli inducing apoptosis to be used in the assay method of the present invention are concerned, all types of stimuli capable of inducing apoptosis on primary cultured cells are included, for: example, deprivations of serum, growth factor and hormone, addition of hormones including glucocorticoids, local chemical mediators including prostaglandins, cytokines including tumor necrosis factor, physiological substances including glutamic acid and bilirubin, or Fas antibody, reagents generating active oxygen species including hydrogen peroxide, NO generating reagents including SNP, anti-cancer drugs including etoposide, staurosporine (for example, *J. Cell Biol*., Vol. 133, p. 1053, 1996), and other various cell intoxication substances, and physical stimuli including radiation and heat.

The present invention will be explained in detail in the following sections by way of Examples but the present invention is, needless to say, not limited to the Examples.

### Examples

### Test Example 1

Porcine ovarian granulosa cells (POGC) were isolated from porcine ovaries and cultured following to a known method (*Endocrinology*, Vol. 136, p. 3470, 1995). Namely, porcine ovaries were rinsed with PBS buffer, and POGC were collected by aspiration from antral follicles with a syringe. Cell fraction was recovered as a precipitate by centrifugation of the suspension. The operation of suspending the cell fraction into PBS buffer and subjecting the resulting suspension to centrifugation was repeated three times to wash the cells. The POGC obtained as a precipitate were suspended again into a culture medium (DMEM containing 10% fetal bovine serum) and cell clumps were disrupted by pipetting. The cell suspension was passed through a mesh to remove contaminating tissue fragments, and then pipetted in 24-well plates for cell culture. The cell suspension was cultured for 2 days in a CO₂ incubator according to a conventional procedure (37°C, 5% CO₂). Then, the medium was refreshed to remove floating cells and the culture was continued for further 2 to 3 days until the cells reached subconfluency (0.7 to 2 × 10⁵ cell/well). After the attaching cells were washed with serum-free medium, they were cultured in a serum-free DMEM (containing 5 µg/mL of transferrin, and 40 ng/mL of hydrocortisone, 4 mg/mL of bovine serum albumin (BSA), 100 nM androstendione).

Addition of SNP (sodium nitroprusside, Na₂(Fe(CN)₅NO), 0.5 mM) known as an NO generating reagent to the POGC resulted in death of all the cells with morphological change characteristic of apoptosis which was found on an observation after 6 hours under a light microscopy and an electron microscopy. The cell death was also confirmed by MTT assay. Then, various concentrations of the test compounds were added to the present culture system and the cells were observed after 48 hours. The results are shown in Table 1 where a minimum effective concentration of each compound for complete inhibition of apoptosis is described.

**Table 1**

| Inhibiting Effects on Apoptosis of Porcine Ovarian Granulosa Cells by SNP stimulation | |
|---|---|
| Compound | Minimum effective concentration (µM) |
| Compound 1* | 15 |
| Compound 2 | 10 |
| Compound 3 | 3 |
| Compound 4 | 10 |
| Compound 5 | 15 |
| Compound 6 | 3 |
| Compound 7 | 7 |
| Compound 8 | 7 |
| Compound 9* | 15 |
| Compound 10** | 3 |

| | |
|---|---|
| * judged at 18 hours after SNP treatment | |
| ** judged at 35 hours after SNP treatment | |

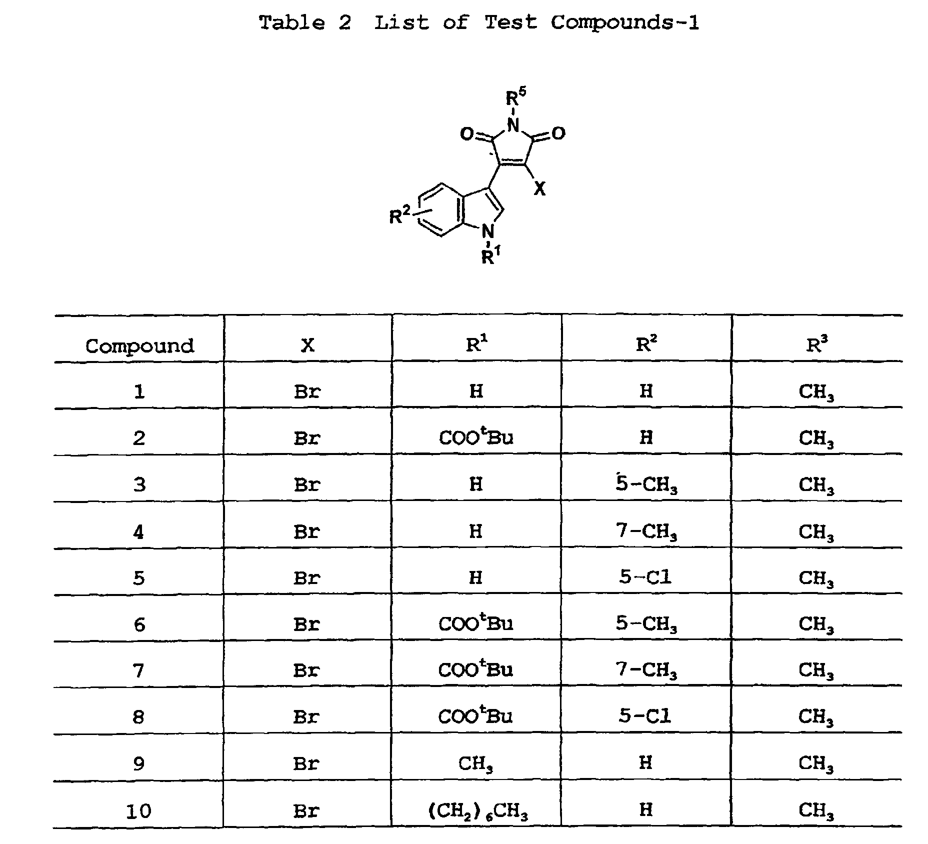

### Referential Example 1

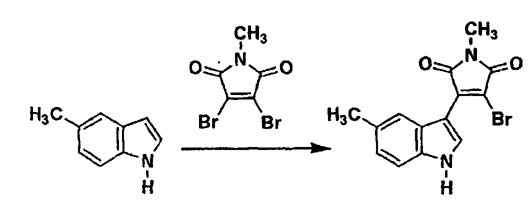

To a solution of 5-methylindole (150 mg, 1.15 mmol) dissolved in THF (3 mL) was added 0.96 M ethylmagnesium bromide (1.20 mL, 1.15 mmol) at 40°C, and the whole was stirred at 40°C for 30 minutes. The reaction mixture was cooled to room temperature and added with a solution of 2,3-dibromo-N-methylmaleimide (155 mg, 0.575 mmol) prepared following to a known method (*Chem. Ber*., p. 764, 1964) and dissolved in THF (5 mL), followed by stirring at room temperature over night. After 20% aqueous citric acid solution (0.5 mL) was added thereto under ice cooling and the whole was stirred, THF was removed by concentration under reduced pressure and the resulting concentrate was extracted with dichloromethane. The extract was dried over magnesium sulfate and then concentrated under reduced pressure. The residue was purified by column chromatography over silica gel (ethyl acetate : n-hexane = 1 : 10 to 1 : 3) to obtain Compound 3 (185 mg, quantitative) as brown solids.
mp 158-159°C
¹H-NMR (CDCl₃) δ 2.51 (s, 3H), 3.18 (s, 3H), 7.13 (d, J=8.4Hz, 1H), 7.33 (d, J=8.4Hz, 1H), 7.82 (s, 1H), 7.96 (d, J=3.0Hz, 1H), 8.66 (brs, 1H)
IR (KBr) 3360, 1690, 1590, 1420, 1370, 1150, 1090, 800, 730, 600 cm⁻¹
MS m/z 318 (M⁺)

### Referential Example 2

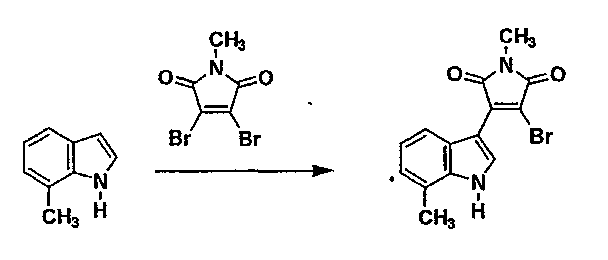

To a solution of 7-methylindole (153 mg, 1.17 mmol) dissolved in THF (3 mL) was added 0.96 H ethylmagnesiuin bromide (1.22 mL, 1.17 mmol) at 40°C, and the whole was stirred at 40°C for 30 minutes. The reaction mixture was cooled to room temperature and added with a solution of 2,3-dibromo-N-methylmaleimide (158 mg, 0.588 mmol) dissolved in THF (5 mL), followed by stirring at room temperature for 2 hours. After 20% aqueous citric acid solution (0.5 mL) was added thereto under ice cooling and the whole was stirred, THF was removed by concentration under reduced pressure and the resulting concentrate was extracted with dichloromethane. The extract was dried over magnesium sulfate and then concentrated under reduced pressure. The residue was purified by column chromatography over silica gel (ethyl acetate : n-hexane = 1 : 10 to 1 : 3) to obtain Compound 4 (171 mg, 91%) as red solids.
mp 153-156°C
¹H-NMR (CDCl₃) δ 2.54 (s, 3H), 3.17 (s, 3H), 7.1-7.2 (m, 2H), 7.88 (d, J=7.6Hz, 1H), 7.99 (d, J=5.0Hz, 1H), 8.69 (brs, 1H)
IR (KBr) 3320, 1690, 1430, 1380, 1150 cm⁻¹
MS m/z 318 (M⁺)

### Referential Example 3

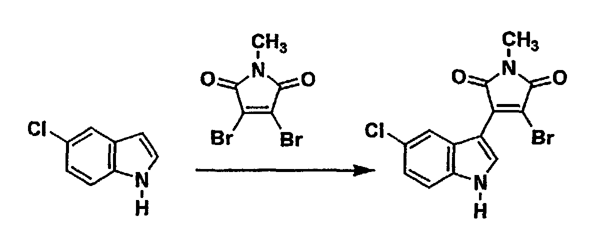

To a solution of 5-chloroindole (153 mg, 1.01 mmol) dissolved in THF (3 mL) was added 0.96 M ethylmagnesium bromide (1.05 mL, 1.01 mmol) at 40°C, and the whole was stirred at 40°C for 30 minutes. The reaction mixture was cooled to room temperature and added with a solution of 2,3-dibromo-N-methylmaleimide (136 mg, 0.505 mmol) dissolved in THE (5 mL), followed by stirring at room temperature for 2 hours. After 20% aqueous citric acid solution (0.5 mL) was added thereto under ice cooling and the whole was stirred, THF was removed by concentration under reduced pressure and the resulting concentrate was extracted with dichloromethane. The extract was dried over magnesium sulfate and then concentrated under reduced pressure. The residue was purified by column chromatography over silica gel (ethyl acetate : n-hexane = 1 : 10 to 1 : 3) to obtain Compound 5 (146 mg, 85%) as pale brown solids.
mp 227-229°C (decomp.)
¹H-NMR (CDCl₃) δ 3.18 (s, 3H), 7.2-7.3 (m, 1H), 7.38 (d, J=7.2Hz, 1H), 8.0-8.1 (m, 2H), 8.78 (brs, 1H)
IR (KBr) 3350, 1690, 1600, 1410, 1370, 610 cm⁻¹
MS m/z 338 (M⁺)

### Referential Example 4

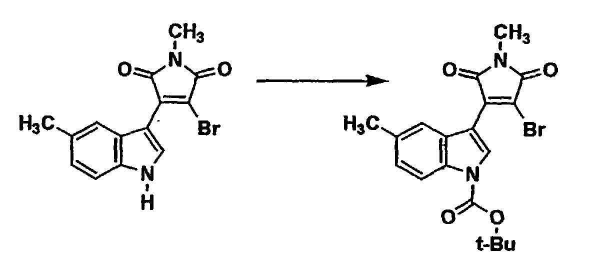

To a solution of Compound 3 (118 mg, 0.370 mmol) dissolved in THF (5 mL) were added di-tert-butyl dicarbonate (97 mg, 0.44 mmol) and dimethylaminopyridine (2.3 mg, 0.019 mmol) at 4°C, and the whole was stirred at 4°C for 1 hour. The reaction mixture was concentrated under reduced pressure and the residue was purified by column chromatography over silica gel (ethyl acetate : n-hexane = 1 : 10) to obtain Compound 6 (75 mg, 48%) as yellow solids.
mp 76-79°C
¹H-NMR (CDCl₃) δ 1.69 (s, 9H), 2.47 (s, 3H), 3.19 (s, 3H), 7.21 (d, J=8.4Hz, 1H), 7.60 (s, 1H), 8.08 (d, J=8.4Hz, 1H), 8.14 (s, 1H)
IR (KBr) 1700, 1430, 1360, 1240, 1140, 1060 cm⁻¹
MS m/z 418 (M⁺)

### Referential Example 5

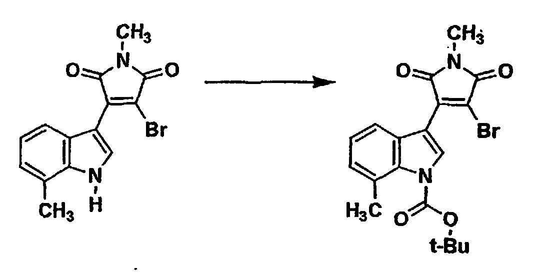

To a solution of Compound 4 (100 mg, 0.313 mmol) dissolved in THF (4 mL) were added di-tert-butyl dicarbonate (82 mg, 0.38 mmol) and dimethylaminopyridine (1.9 mg, 0.016 mmol) at 4°C, and the whole was stirred at 4°C for 1 hour and further at room temperature for 1 hour. The reaction mixture was concentrated under reduced pressure and the residue was purified by column chromatography over silica gel (ethyl acetate : n-hexane = 1 : 10 to 1 : 1) to obtain Compound 7 (139 mg, quantitative) as yellow solids.
mp 54-57°C
¹H-NMR (CDCl₃) δ 1.67 (s, 9H), 2.66 (s, 3H), 3.18 (s, 3H), 7.1-7.3 (m, 2H), 7.59 (m, 1H), 8.05 (s, 1H)
IR (KBr) 2960, 1710, 1610, 1430, 1380, 1210, 1140, 1040, 850, 740 cm⁻¹
MS m/z 418 (M⁺)

### Referential Example 6

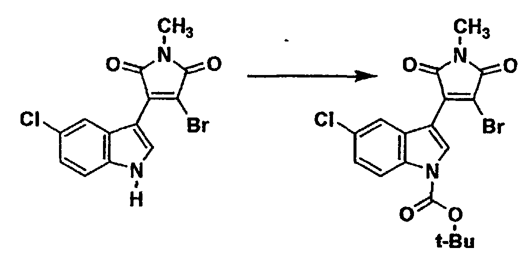

To a solution of Compound 5 (88 mg, 0.259 mmol) dissolved in THF (3.5 mL) were added di-tert-butyl dicarbonate (68 mg, 0.31 mmol) and dimethylaminopyridine (1.6 mg, 0.013 mmol) at 4°C, and the whole was stirred at 4°C for 1 hour. The reaction mixture was concentrated under reduced pressure and the residue was purified by column chromatography over silica gel (ethyl acetate : n-hexane = 1 : 10) to obtain Compound 8 (89 mg, 78%) as yellow solids.
mp 155-157°C
¹H-NMR (CDCl₃) δ 1.72 (s, 9H), 3.20 (s, 3H), 7.36 (dd, J=1.2, 8.4Hz, 1H), 7.83 (d, J=1.2Hz, 1H), 8.14 (d, J=8.4Hz, 1H), 8.21 (s, 1H)
IR (KBr) 1700, 1510, 1440, 1360, 1260, 1230, 1200, 1140, 1060, 1000, 820, 750, 700 cm⁻¹
MS m/z 438 (M⁺)

### Referential Example 7

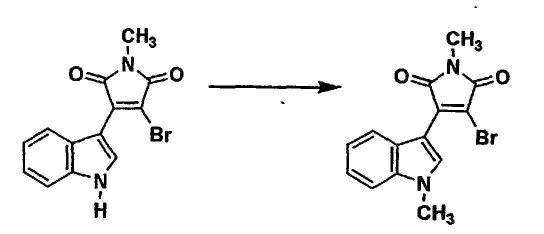

To a solution of Compound 1 (100 mg, 0.327 mmol) synthesized following to a known method and dissolved in acetone (10 mL) were added potassium carbonate (49.8 mg, 0.360 mmol) and dimethyl sulfate (0.04 mL, 0.43 mmol) successively, and the whole was stirred under heating and refluxing over night. The reaction mixture was added with water (5 mL) and, concentrated under reduced pressure to remove acetone. After the concentrate was extracted with dichloromethane, the extract was dried over magnesium sulfate, and then concentrated under reduced pressure. The residue was purified by column chromatography over silica gel (ethyl acetate : n-hexane = 1 : 10 to 1 : 3) to obtain Compound 9 (89 mg, 85%) as pale brown solids.
mp 155-158°C
¹H-NMR (CDCl₃) δ 3.17 (s, 3H), 3.89 (s, 3H), 7.2-7.4 (m, 3H), 7.90 (s, 1H), 8.06 (d, J=7.6Hz, 1H)
IR (KBr) 1770, 1700, 1580, 1510, 1430, 1370, 1230, 1150, 1120, 980, 800, 730 cm⁻¹
MS m/z 318 (M⁺)

### Referential Example 8

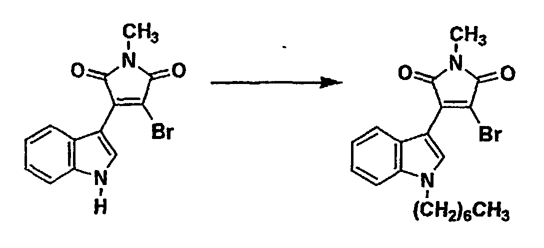

A DMF solution (2 mL) of Compound 1 (130 mg, 0.425 mmol) synthesized following to a known method was added to sodium hydride (oily, 60 to 72%, 34 mg) suspended in DMF (0.5 mL), and the whole was stirred at room temperature for 30 minutes. The reaction mixture was added with n-heptyl bromide (0.60 mL, 4.3 mmol) and then stirred at 40°C for 1.5 hours. After the reaction mixture was concentrated under reduced pressure to remove DMF, the concentrate was added with water (50 mL) and extracted with dichloromethane (200 mL × 2) and ethyl acetate (100 mL × 1). The organic layers were combined. The combined extract was washed with water (50 mL × 2) and then dried over magnesium sulfate. After the solvent was removed under reduced pressure, the residue was purified by column chromatography over silica gel (ethyl acetate : n-hexane = 1 : 10 to 1 : 1) to obtain Compound 10 (39 mg, 23%) as a brown oily substance.
¹H-NMR (CDCl₃) δ 0.8-2.0 (m, 13H), 3.16 (s, 3H), 4.20 (t, J=7.2Hz, 2H), 7.2-7.5 (m, 3H), 7.95 (s, 1H), 8.08 (m, 1H)
IR (KBr) 2950, 2880, 1770, 1710, 1620, 1510, 1440, 1390, 1210, 1180, 1130, 1020, 980, 840, 810, 740 cm⁻¹
MS m/z 402 (M⁺)

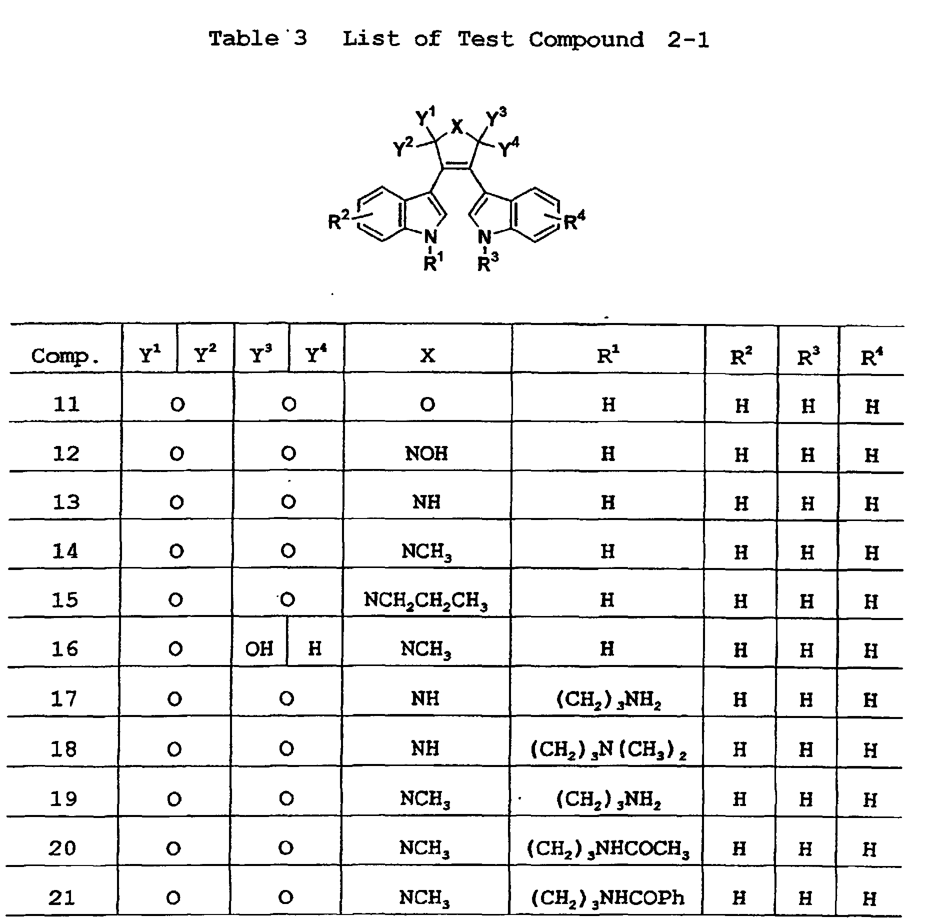

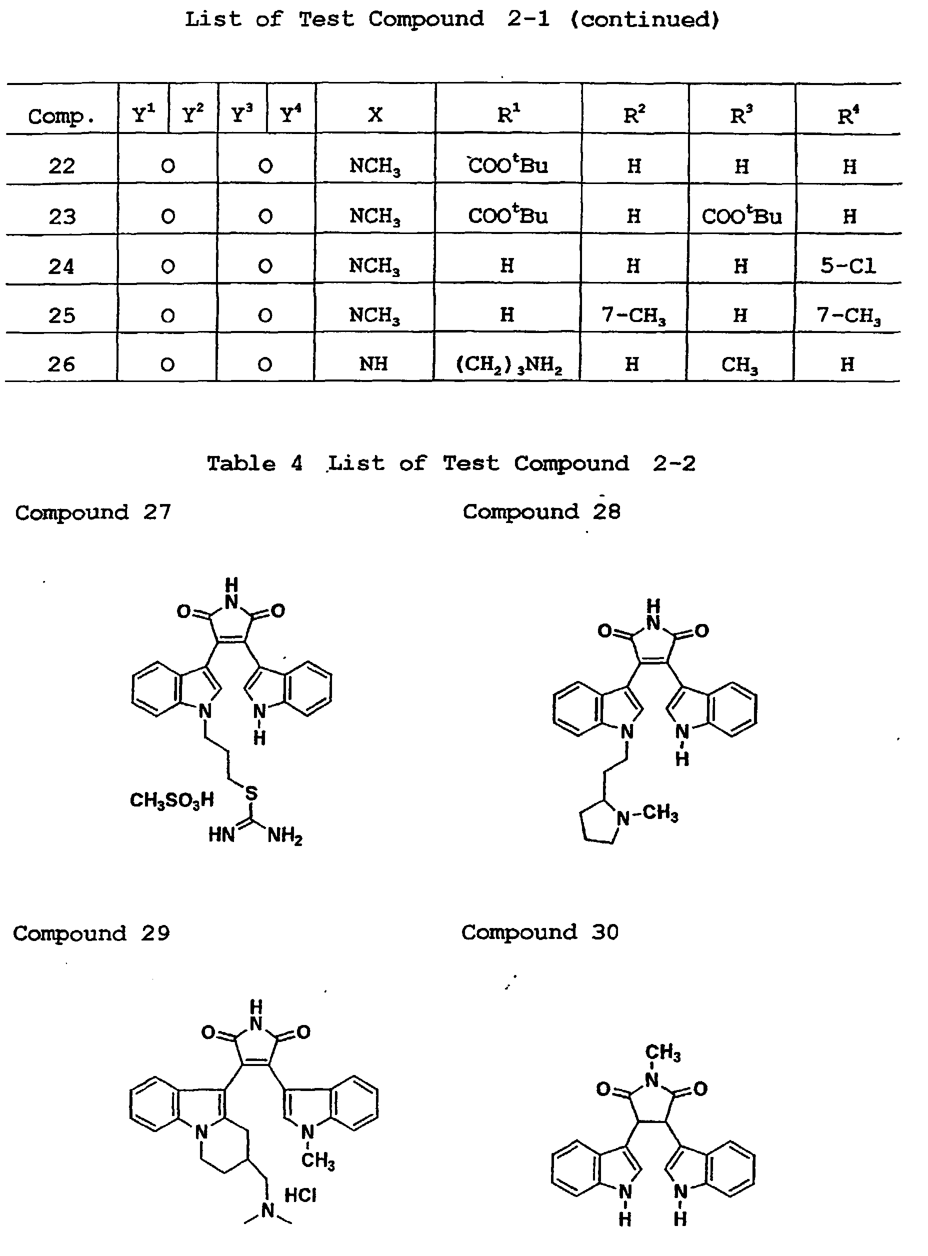

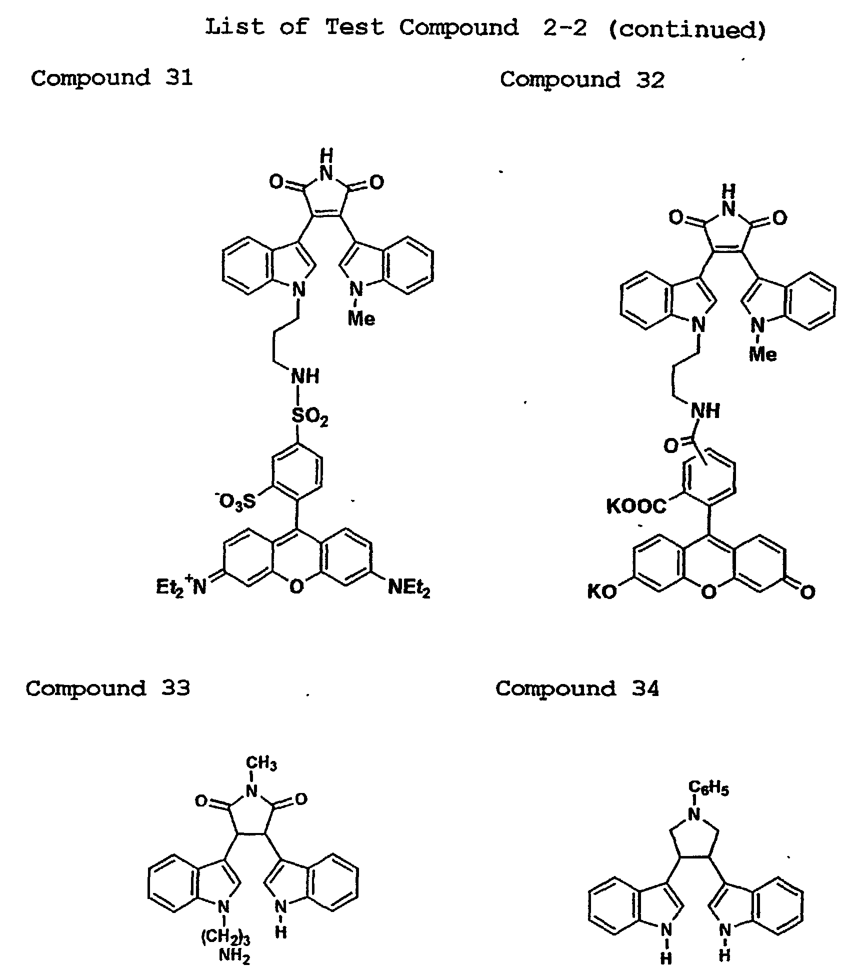

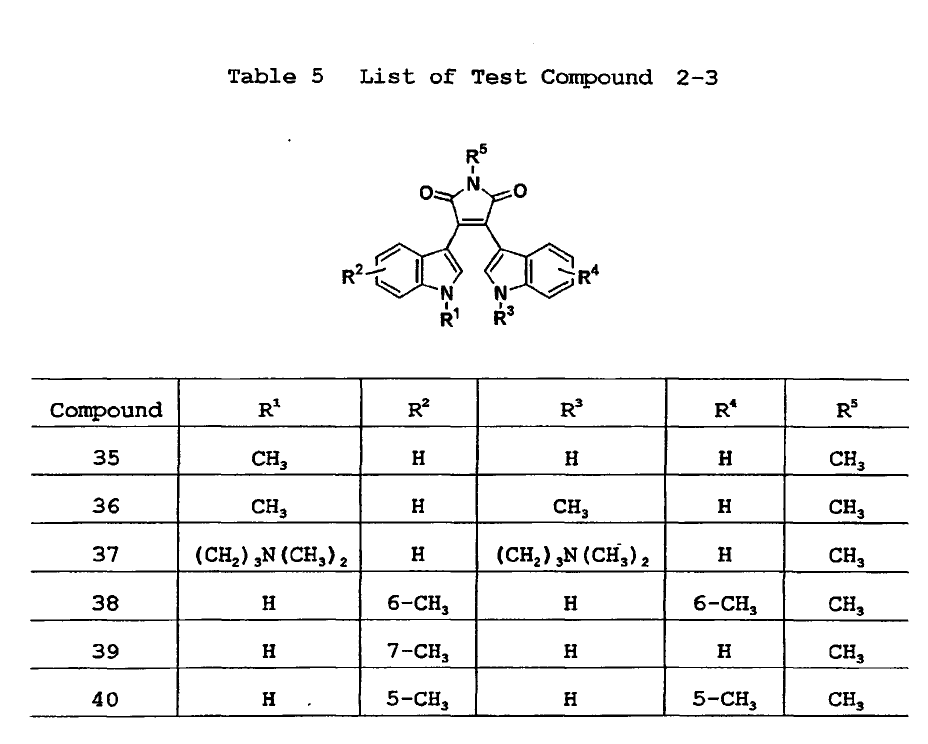

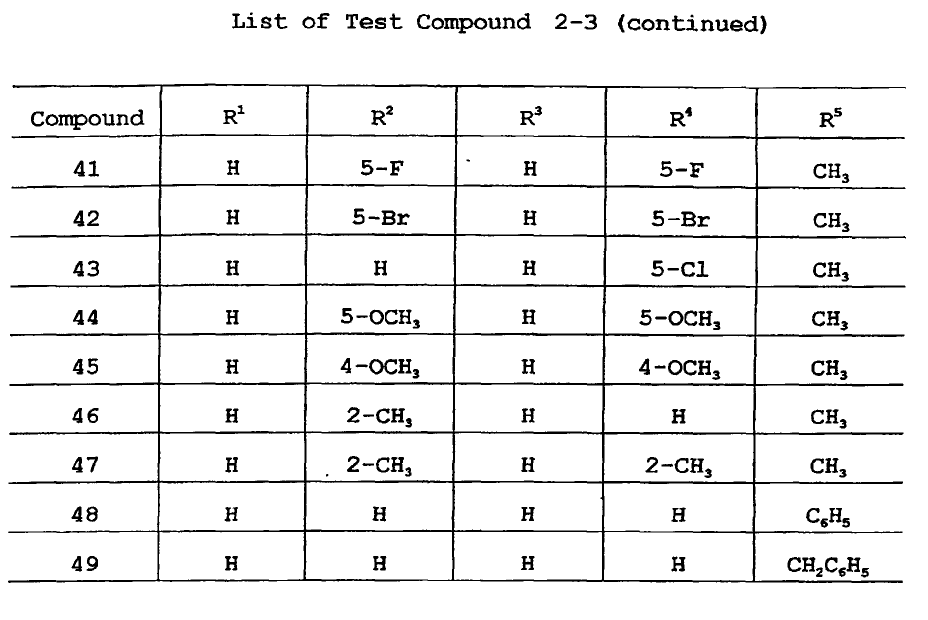

### Test Example 2

The ovarian granulosa cells are cells surrounding an ovum, and proliferate and differentiate by the action of follicle stimulating hormone (FSH) and luteinizing hormone (LH) in response to sexual cycles, during which they acquire a progesterone-producing ability. Unovulatory follicles finally undergo apoptosis and disappear. The differentiation process can be mimicked following to a known method (*Endocrinology*, Vol. 136, p. 3470, 1995) as shown in the following *in vitro* experiment. Furthermore, when they are continuously cultured with maintaining final differentiation ability, they are observed to undergo apoptosis as described below. Namely, porcine ovaries were rinsed with PBS buffer, and porcine ovarian granulosa cells (POGC) were collected by aspiration from antral follicles with a syringe. Cell fraction was recovered as a precipitate by centrifugation of the suspension. The operation of suspending the cell fraction into PBS buffer and subjecting the resulting suspension to centrifugation was repeated three times to wash the cells. The POGC obtained as a precipitate were suspended again into a culture medium (DMEM containing 10% fetal bovine serum) and cell clumps were disrupted by pipetting. The cell suspension was passed through a mesh to remove contaminating tissue fragments, and then pipetted in 24-well plates for cell culture. The cell suspension was cultured for 2 days in a CO₂ incubator according to a conventional procedure (37°C, 5% CO₂). Then, the medium was refleshed to remove floating cells and the culture was continued for further 2 to 3 days until the cells reached subconfluency (0.7 to 2 × 10⁵ cell/well). After the attaching cells were washed with serum-free medium, they were cultured in serum-free DMEM added with FSH and LH (containing 5 µg/mL of transferrin, and 40 ng/mL of hydrocortisone, 4 mg/mL of bovine serum albumin (BSA), 100nM androstendione, and 100 ng/mL of FSH and 10 ng/mL of LH) for additional 3 days, and, after replacement of the medium by new medium having the same composition, the culture was continued. Such treatment induced terminal differentiation with the cell shape change from a fibroblastic conformation into an epithelioid one and with showing an active hormone-producing ability. Upon continued culture, these completely differentiated cells undergo cell death synchronously after about 5 days, the cell death being monitored by a trypan blue exclusion test and a MTT (3-(4,5-dimethylthiazol-2-yl)-2,5-diphenyltetrazolium bromide) test. Observation on light microscopy and electron microscopy revealed that the cell death shared morphological change of typical apoptosis. When Test Compound 18 was added at a concentration of 3 µM into the above cultures on 4 days after the final medium refreshment, more than 95% of cells were found to be alive on an observation even after 7 days from the addition of the test compound.

### Test Example 3

When prostaglandin F₂α (PGF₂α) (50 µg/mL) was added to the terminally differentiated cells as described in Test Example 2 after 2 days from the final refreshment of culture medium, cell death was observed on light microscopy and electron microscopy after 16 hours with morphological change characteristic of apoptosis. However, when Test Compound 18 (3 µM) was added into the above medium prior to the PGF₂α addition and culture was continued under the same conditions, cell death was inhibited and observation after 16 hour revealed survival of more than 95% of the cells.

### Test Example 4

POGC were isolated and cultured until subconfluency as described in Test Example 2. When the cells, after being washed with serum-free medium, were cultured in a FSH-, LH-and serum-free medium (containing 5 µg/mL of transferrin, 40 ng/mL of hydrocortisone, 4 mg/mL of BSA, and 100nM androstendione) in the absence of hormonal stimulation, culture was able to continue with relatively less differentiation as compared with the cells in the presence of FSH and LB (Test Example 2). A long term culture (2 to 3 weeks) in this serum-free medium lead to cell death with morphological change characteristic of apoptosis under a light microscopy. This cell death occurred non-synchronously and slowly, and it, took about one week to complete the cell death. However, when Test Compound 18 (3 µM) was added into the above medium, cell death was inhibited and an observation at 7 days after the death of the cells treated with no test compound revealed survival of more than 95% of the cells.

### Test Example 5

POGC isolated, cultured, and reached subconfluency as described in Test Example 2 were washed with a serum-free medium. Then, the cells were cultured in a serum-containing medium with FSH and LB (containing 5 µg/mL of transferrin, and 40 ng/mL of hydrocortisone, 4 mg/mL of BSA, 100nM androstendione, and 100 ng/mL of FSH and 10 ng/mL of LH) to obtain completely differentiated cells. Addition of staurosporine (3 nM) thereto resulted in death of all the cells with morphological change characteristic of apoptosis, which was observed under light microscopy after 44 hours. Then Test Compound 18 (3 µM) was added into the above medium prior to the addition of staurosporine and culture was continued under the same conditions, cell death was inhibited and more than 95% of the cells were alive on an observation even after 68 hours.

### Test Example 6

It was observed under light microscopy that addition of hydrogen peroxide (100 µm) into undifferentiated POGC cultured as described in Test Example 4 resulted in death of all the cells after 12 hours with morphological change characteristic of apoptosis. Then Test Compound 18 (3 µM) was added into the above medium prior to the addition of hydrogen peroxide and culture was continued under the same conditions, cell death was completely inhibited and more than 95% of the cells were alive on an observation even after 24 hours.

### Test Example 7

Addition of SNP (sodium nitroprusside, Na₂(Fe(CN)₅NO), 0.5 mM) known as an NO generating reagent to undifferentiated POGC cultured as described in Test Example 4 resulted in death of all the cells with morphological change characteristic of apoptosis which was found on an observation after 6 hours under a light microscopy and an electron microscopy. The cell death was also confirmed by MTT assay. Then, various concentrations of the test compounds were added to the culture system and the cells were observed after 48 hours. The results are shown in Table 6 where the minimum effective concentration of each compound for complete inhibition of apoptosis is described.

**Table 6**

| Inhibiting Effects on Apoptosis of Porcine Ovarian Granulosa Cells by SNP stimulation | | | |
|---|---|---|---|
| Compound | Minimum effective concentration (µM) | Compound | Minimum effective concentration (µM) |
| 11* | 10 | 31* | 3 |
| 12 | 15 | 32 | 3 |
| 13 | 5 | 33** | 10 |
| 14 | 5 | 34** | 0.3 |
| 15 | 3 | 35** | 10 |
| 16 | 5 | 36** | 10 |
| 17* | 3 | 37** | 10 |
| 18 | 0.7 | 38** | 0.3 |
| 19 | 0.7 | 39** | 3 |
| 20 | 7 | 40** | 1 |
| 21 | 7 | 41** | 10 |
| 22 | 5 | 42** | 1 |
| 23 | 5 | 43** | 3 |
| 24 | 3 | 44** | 1 |
| 25 | 0.3 | 45** | 3 |
| 26 | 1 | 46** | 10 |
| 27 | 1 | 47** | 10 |
| 28** | 3 | 48** | 1 |
| 29** | 1 | 49** | 1 |
| 30 | 10 | | |

| | | | |
|---|---|---|---|
| * judged at 30 hours after SNP treatment | | | |
| ** judged at 18 hours after SNP treatment | | | |

### Comparative Example 1

Inhibitory action on apoptosis of olomoucine (purchased from Calbiochem-Novabiochem corporation) known as a cycline-dependent kinase inhibitor was tested according to the method as described in Test Example 7. The result showed that it has no inhibitory action on apoptosis even at a concentration of as high as 30 µM.

### Test Example 8

Addition of bilirubin (30 µg/mL) to undifferentiated POGC cultured as described in Test Example 4 resulted in cell death: 50% of cell death after 2 days and 95% of cell death after 3 days under a light microscopy. The cell death was also confined by a trypan blue exclusion test and a MTT assay. When Test compound 14 (10 µM) or Test Compound 25 (10 µM) was added into the above medium prior to the addition of bilirubin and culture was continued under the same conditions, cell death was inhibited and observations even after 4 days revealed survival of 50% or 95% of the cells, respectively.

### Test Example 9

Mouse granulosa cells were transfected with SV40 T antigen and cDNA of steroidogenic factor-1 (SF-1/Ad4BP) to establish a cell line, named 4B2. The cell secretes progesterone in response to protein kinase-A. The 4B2 cells were cultured in 48-well culture plates until subconfluency in DMEM containing 10% fetal bovine serum in a CO₂ incubator according to a conventional manner. After the medium was switched to DMEM containing 2% fetal bovine serum, SNP (0.5 mM) was added thereto. Observation after 16 hours revealed death of all the cells with morphological change characteristic of apoptosis, under a light microscopy and an electron microscopy. Then, prior to the addition of SNP, Test Compound 13, 14, 17, 20, 24 or 25 (10 µM each) was added to the present medium and observations on cells were made after 24 hours. As a result, addition of any of the compounds completely inhibited apoptosis and more than 95% of the cells were alive.

### Test Example 10

According to a known method (*A Dissection and Tissue Culture Manual of the Nervous System,* p. 211, 1989, Alan R, Liss, Inc.), cerebellar granule cells were isolated from the cerebellum of 7 days old rats and cultured. Namely, after conducting isolation procedures described in the above literature, the resulting cells were resuspended in DMEM containing 10% fetal bovine serum, 25 mM KCl and 2 mM glutamine, and seeded in poly-lysine coated plates. After the cells were cultured in a CO₂ incubator for 48 hours according to a conventional method, cytosine-1-β-D(+)-arabinofuranoside (Ara-C) (10 µM) was added thereto, and the cell was continued to culture. The following experiments were conducted with the cells of 7 to 14 days old from the start day of the culture, the cells having completed neurite extension sufficiently. Addition of hydrogen peroxide (10 µM) to the cerebellar granule cells cultured as above resulted in death of all the cells, which was observed after 12 hours under a light microscopy. When Test Compound 19 (10 µM) or Test Compound 25 (10 µM) was added to the medium prior to the addition of hydrogen peroxide, observation similarly after 12 hours revealed that more than 95% of the cells were alive. Furthermore, experiments were conducted by increasing a concentration of hydrogen peroxide to 300 µM. In the cases that Test Compound 18 (10 µM), Test Compound 19 (10 µM), or Test Compound 20 (10 µM) were added, about 90%, 90%, and 70% of the cells were alive, respectively, on observations after 16 hours.

### Test Example 11

When SNP (1.5 µM) was added to the cerebellar granule cells cultured as described in Test Example 10, all the cells were found to be dead on an observation after 12 hours under a light microscopy. In the cases that Test Compound 14 (10 µM), Test Compound 19 (10 µM), Test Compound 20 (10 µM), or Test Compound 25 (10 µM) was added prior to the SNP addition, more than 95% of cells were alive on observations after 12 hours.

### Test Example 12

Neonatal jaundice is known to induce severe brain damages by way of abnormally high concentration of bilirubin in the body. This is likely caused by neuronal cell damages due to bilirubin. In fact, when bilirubin (100 µg/ml) was added to cerebellar granule cells cultured as described in Test Example 10, occurrence of cell death was observed under a light microscopy, and all cells were found to be dead on an observation after 18 hours. In the case that Test Compound 20 (10 µM) was added prior to the bilirubin addition, about 80% of cells were alive on an observation after 18 hours.

### Test Example 13

After human blood (2 mL) was centrifuged at 1000 rpm for 5 min, the resulting cell pellets containing erythrocytes were washed three times with a DMEM medium and the cells were resuspended in the same medium (15 mL) (about 5 × 10⁸ cells/mL). Ten µL of this suspension was added with 10% FCS/DMEM (10 mL), and the cells were plated in 48 well plates (500 µL/well). When hydrogen peroxide (300 µM) was added to the erythrocyte culture solution and the cells were cultured for 24 hours, all erythrocytes were dead on observations under a light microscopy (all erythrocytes were subjected to shape changes from a bright, round and concave shape into a dark hemolytic ghost through multiple shape changes). On the other hand, Test Compound 14 (30 µM), Test Compound 18 (10 µM), Test Compound 19 (20 µM) or Test Compound 20 (20 µM) was added prior to the hydrogen peroxide addition. In these cases, observations made similarly after 24 hours showed that the ratios of dead erythrocytes (dark ghost cells) decreased, greatly as compared with the case of hydrogen peroxide alone, to 20%, 20%, 10% or 10%, respectively.

### Test Example 14

Primary cultured fibroblasts were prepared from mice according to a known method (*Kinou Saibou no Bunri to Baiyou* (*Isolation and Culture of Functional Cells*), Maruzen, 1987, p. 59). Namely, after removing skin hairs from mice with shaving foam followed by disinfection with a chlorhexidine gluconate solution, tiny skin fragments were isolated. These fragments were placed under a slide glass so that they did not float in a culture dish and cultured in DMEM containing 10% fetal bovine serum in a CO₂ incubator according to a conventional manner. After migration of fibroblasts became obvious, the slide glasses and the residual skin fragments were removed, and the culture was continued. Then SNP (0.5 mM), known as an NO generator, was added to the fibroblasts, all cells were found to be dead on an observation after 12 hours, with showing morphological change characteristic of apoptosis under a light microscopy and an electron microscopy. In the cases that Test Compound 14 (5 µM) or Test Compound 18 (2.5 µM) was added to the present culture system prior to the SNP addition, apoptosis in both cultures was inhibited and more than 95% of cells were alive on an observation after 48 hours.

### Test Example 15

Kidneys isolated from mice were dissected into minute pieces in a serum-free DMEM, which were dispersed with trypsin/EDTA treatment. After leaving it for a while, the supernatant was discarded, and the pellet was transferred to a culture plate and cultured in DMEM containing 10% fetal bovine serum in a CO₂ incubator according to a conventional manner. After tissues were adhered onto a culture plate and subsequently cell colonies got formed, the tissues were removed and the colonies were subcultured in the same medium. The resulting monolayer cells derived from the mouse kidneys were morphologically different from fibroblasts. When SNP (0.5 mM), known as an NO generator, was added to the cells, it was found that all cells were dead with showing morphological change characteristic of apoptosis on an observation after 12 hours under a light microscopy. Then, Test Compound 14 (5 µM) or Test Compound 18 (2.5 µM) was added prior to the SNP addition and the cells were observed. The results showed that apoptosis was inhibited in both cases on observations after 48 hours, and more than 95% of the cells were found to be alive.

### Referential Example 9

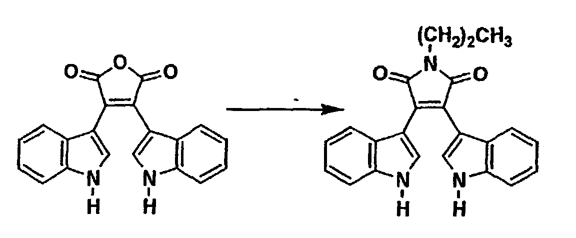

To a solution of Compound 11 (46 mg, 0.14 mmol) synthesized following to a known method *(Tetrahedron*, Vol. 44, p. 2887, 1988) and dissolved in DMF (8 mL) and water (8 mL) was added n-propylamine (0.06 mL, 0.6 mmol), and the whole was stirred at 100°C for 1 hour. The reaction mixture was concentrated under reduced pressure to remove DMF, and the concentrate was extracted with ethyl acetate. The extract was washed with water, dried over magnesium sulfate, and then concentrated under reduced pressure. The residue was purified by column chromatography over silica gel (ethyl acetate : n-hexane = 1 : 3) to obtain Compound 15 (45 mg, 87%) as red solids.
mp 116-119°C
¹H-NMR (CD₃OD) δ 0.97 (t, J=7.6 Hz, 3H), 1.71 (tq, J=7.6, 7.6 Hz, 2H), 3.61 (t, J=7.6Hz, 2H), 6.58 (m, 2H), 6.77 (m, 2H), 6.95 (m, 2H), 7.23 (m, 2H), 7.65 (s, 2H)
IR (KBr) 3380, 1680, 1530, 1400, 1240, 740 cm⁻¹
MS m/z 369 (M⁺)

### Referential Example 10

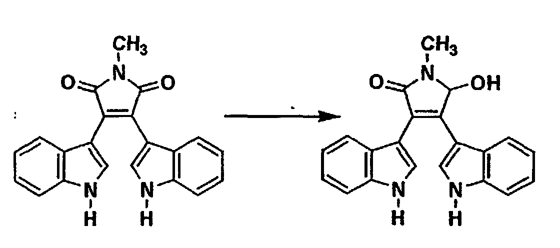

To a solution of Compound 14 (163 mg, 0.477 mmol) synthesized following to a known method (*Tetrahedron*, Vol. 44, p. 2887, 1988) and dissolved in THF (10 mL) was added lithium aluminum hydride (27 mg, 0.72 mmol), and the whole was stirred at room temperature for 29 hours. The reaction mixture was added with water (8 mL) and rendered pH 2 by means of 2N aqueous hydrochloric acid. After removal of THF by concentration under reduced pressure, the concentrate was extracted with dichloromethane and ethyl acetate. The extract was dried over magnesium sulfate and then concentrated under reduced pressure. The residue was purified by column chromatography over silica gel (ethyl acetate : n-hexane = 1 : 2 to 5 : 1) to obtain Compound 16 (87 mg, 53%) as pale brown solids.
mp > 280°C
¹H-NMR (CD₃OD) δ 3.12 (s, 3H), 5.93 (s, 1H), 6.6-6.7 (m, 2H), 6.9-7.4 (m, 8H)
IR (KBr) 3400, 1660, 1540, 1410, 1240, 1090, 1050, 740 cm⁻¹
MS m/z 343 (M⁺)

### Referential Example 11

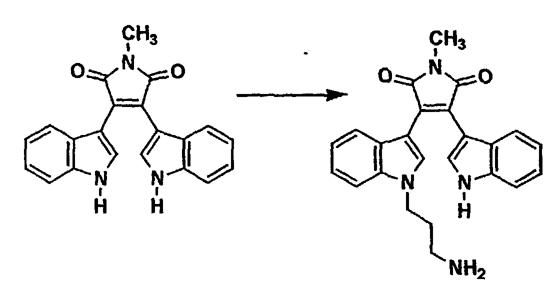

Sodium hydride (60 to 72%, oily, 144 mg) was washed with pentane and then suspended into DMF (0.8 mL). A DMF solution (3 mL) of Compound 14 (409 mg, 1.2 mmol) synthesized following to a known method (*Tetrahedron*, Vol. 44, p. 2887, 1988), and the whole was stirred at room temperature for 45 minutes. On the other hand, DMF (2.0 mL) was added at 0°C to a mixture of 3-chloropropylamine hydrochloride (159 mg, 1.2 mmol) and sodium hydride (60 to 72%, oily, 48 mg) washed with pentane, and the whole was stirred for 5 minutes and then warmed to room temperature with standing. The supernatant was added to the solution of sodium salt of Compound 14, and the residue was extracted with DMF (1 mL) and the supernatant was also added to the solution of sodium salt of Compound 14. The resulting mixture was stirred at 40°C for 1 hour, and then concentrated under reduced pressure to remove DMF. To the residue was added dichloromethane and saturated aqueous sodium chloride solution, and the organic layer was separated. The water layer was extracted three times with dichloromethane. The resulting organic layers were combined and dried over sodium sulfate. The residue obtained by removing the solvent under reduced pressure was purified by column chromatography over silica gel (dichloromethane : isopropylamine = 20 : 1) to obtain Compound 19 (296 mg, 55%) as dark red solids.
mp 137-140°C
¹H-NMR (DMSO-d₆) δ 1.81 (tt, J=6.7, 6.7 Hz, 2H), 3.05 (s, 3H), 3.1-3.5 (m, 2H), 4.09 (brs, 2H), 4.30 (t, J=6.7Hz, 2H), 6.60 (t, J=7.5Hz, 1H), 6.67 (t, J=7.7Hz, 1H), 6.74 (d, J=8.0Hz, 1H), 6.85 (d, J=8.0Hz, 1H), 6.97 (t, J=7.5Hz, 1H), 7.03 (t, J=7.7Hz, 1H), 7.37 (d, J=8.2Hz, 1H), 7.49 (d, J=8.2Hz, 1H), 7.76 (s, 1H), 7.79 (s, 1H), 11.70 (bra, 1H)
IR (KBr) 3350, 2910, 1750, 1680, 1530, 1430, 1380, 740 cm⁻¹
MS m/s 398 (M⁺)

### Referential Example 12

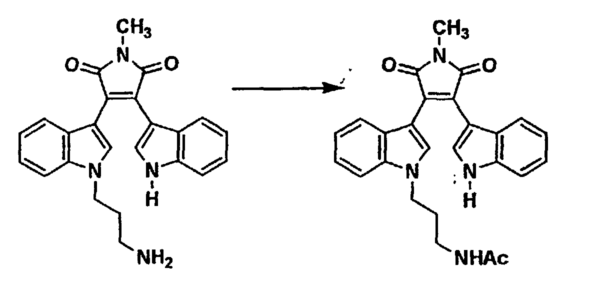

Triethylamine (7 µL, 0.05 mmol) and acetic anhydride (1.1 µL) were added to a dichloromethane solution (300 µL) of compound 19 (4.0 mg, 0.01 mmol), and the whole was stirred at room temperature for 70 minutes. The reaction mixture was added with saturated aqueous sodium bicarbonate solution and then extracted with dichloromethane. The organic layer was dried over sodium sulfate, and the residue obtained by removing the solvent under reduced pressure was purified by column chromatography over silica gel (dichloromethane : ethyl acetate = 1 : 1 to 1 : 2) to obtain Compound 20 (3.2 mg, 72%) as dark red solids.
mp 86-89°C
¹H-NMR (CDCl₃) δ 1.86 (s, 3H), 2.05 (tt, J=6.7, 6.7Hz, 2H), 3.14 (dt, J=6.7, 6.7Hz, 2H), 3.19 (s, 3H), 4.19 (t, J=6.7Hz, 2H), 5.40 (brt, J=6.7Hz, 1H), 6.7-6.8 (m, 2H), 6.99 (d, J=8.1Hz, 1H), 7.0-7.1 (m, 3H), 7.28 (d, J=8.3Hz, 1H), 7.35 (d, J=8.2Hz, 1H), 7.61 (s, 1H), 7.77 (d, J=2.7Hz, 1H), 8.68 (brs, 1H)
IR (KBr) 3370, 1690, 1530, 1430, 1380, 740 cm⁻¹
MS m/z 440 (M⁺)

### Referential Example 13

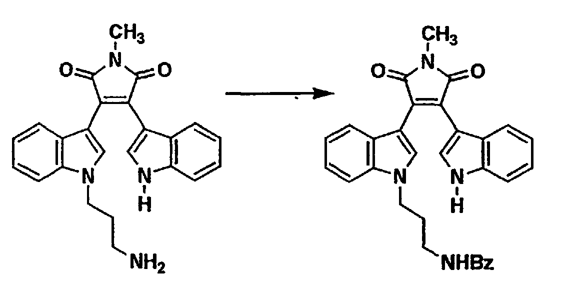

Pyridine (18 µL, 0.23 mmol) and benzoyl chloride (5.2 µL, 0.045 mmol) were added to a dichloromethane solution (1 mL) of Compound 19 (18 mg, 0.045 mmol), and the whole was stirred at room temperature for 2 hours. Pyridine (18 µL, 0.23 mmol) and benzoyl chloride (2.0 µL, 0.017 mmol) were again added thereto, and the whole was further stirred at room temperature for 1 hour. The reaction mixture was added with saturated aqueous sodium bicarbonate solution and then extracted with dichloromethane. The organic layer was dried over sodium sulfate, and the residue obtained by removing the solvent under reduced pressure was purified by column chromatography over silica gel (hexane : ethyl acetate = 1 : 1) to obtain Compound 21 (17.0 mg, 75%) as dark red solids.
mp 130-133°C
¹H-NMR (CDCl₃) d 2.14 (tt, J=6.6, 6.6Hz, 2H), 3.18 (s, 3H), 3.32 (dt, J=6.6, 6.6Hz, 2H), 4.21 (t, J=6.6Hz, 2H), 6.10 (brt, J=6.6Hz, 1H), 6.7-7.8 (m, 15H), 8.71 (brs, 1H)
IR (KBr) 3350, 1750, 1680, 1530, 1420, 1380, 730 cm⁻¹
MS m/z 502 (M⁺)

### Referential Example 14

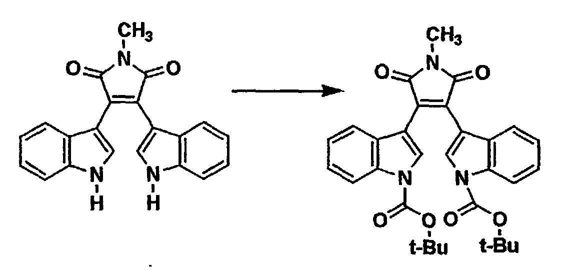

To a solution of Compound 14 (82 mg, 0.24 mmol) dissolved in THF (5 mL) were added di-tert-butyl dicarbonate (52 mg, 0.24 mmol) and dimethylaminopyridine (1.5 mg, 0.012 mmol) at 4°C, and the whole was stirred at 4°C for 1 hour and further at room temperature over night. The reaction mixture was concentrated under reduced pressure and the residue was purified by column chromatography over silica gel (ethyl acetate : n-hexane = 1 : 10 to 1 : 1) to obtain Compound 23 (62 mg, 48%) as yellow solids.
mp 110-113°C
¹H-NMR (CDCl₃) δ 1.68 (s, 18H), 3.22 (s, 3H), 6.8-7.0 (m, 4H), 7.16 (m, 2H), 8.1-8.2 (m, 2H), 8.13 (s, 2H)
IR (KBr) 2960, 1730, 1550, 1440, 1350, 1240, 1140, 1060, 850, 730 cm⁻¹
MS m/z 541 (M⁺)

### Referential Example 15

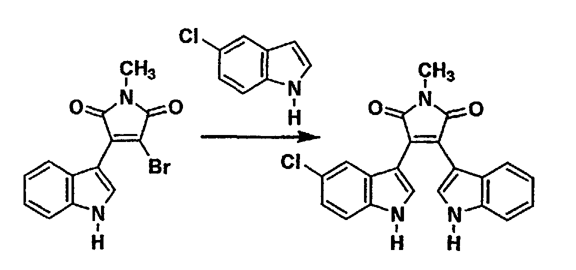

To a solution of 5-chloroindole (122 mg, 0.805 mmol) dissolved in toluene (3 mL) was added 0.96 M ethylmagnesium bromide (0.84 mL, 0.81 mmol) at 40°C, and the whole was stirred at 40°C for 45 minutes. Successively, thereto was added a solution of 2-bromo-3-(1H-indol-3-yl)-N-methylmaleimide (70.1 mg, 0.229 mmol) synthesized following to a known method (*Tetrahedron*, Vol. 44, p. 2887, 1988) and dissolved in toluene (9 mL), followed by stirring under heating and refluxing for 2 hours. After 20% aqueous citric acid solution (0.5 mL) was added thereto under ice cooling and the whole was stirred, toluene was removed by concentration under reduced pressure and the resulting concentrate was extracted with dichloromethane. The extract was dried over magnesium sulfate and then concentrated under reduced pressure. The residue was purified by column chromatography over silica gel (ethyl acetate : n-hexane = 1 : 5 to 1 : 1) to obtain Compound 24 (89 mg, quantitative) as pale brown solids.
mp 114-117°C
¹H-NMR (CDCl₃) δ 3.20 (s, 3H), 6.6-7.4 (m, 7H), 7.76 (m, 1H), 7.86 (m, 1H), 8.50 (brs, 1H), 8.60 (brs, 1H)
IR (KBr) 3350, 1680, 1530, 1440, 1370, 730 cm⁻¹
MS m/z 375 (M⁺)

### Referential Example 16

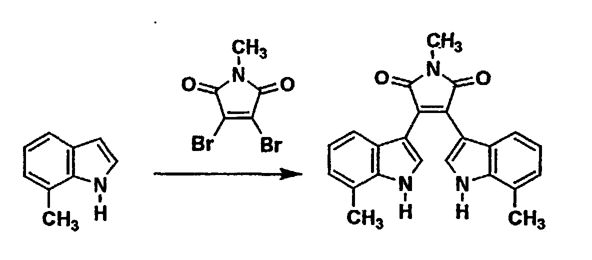

To a solution of 7-methylindole (162 mg, 1.23 mmol) dissolved in toluene (4 mL) was added 0.96 M ethylmagnesium bromide (1.28 mL, 1.23 mmol) at 40°C, and the whole was stirred at 40°C for 45 minutes. Successively, thereto was added a solution of 2,3-dibromo-N-methylmaleimide (70.4 mg, 0.276 mmol) dissolved in toluene (9 mL), followed by stirring under heating and refluxing for 2 hours. After 20% aqueous citric acid solution (0.5 mL) was added thereto under ice cooling and the whole was stirred, toluene was removed by concentration under reduced pressure and the resulting concentrate was extracted with dichloromethane. The extract was dried over magnesium sulfate and then concentrated under reduced pressure. The residue was purified by column chromatography over silica gel (ethyl acetate : n-hexane = 1 : 5 to 1 : 1) to obtain Compound 25 (99 mg, 97%) as red solids.
mp > 300°C
¹H-NMR (CDCl₃) δ 2.48 (s, 6H), 3.21 (s, 3H), 6.6-7.0 (m, 6H), 7.75 (d, J=3.0Hz, 2H), 8.43 (brs, 2H)
IR (KBr) 3320, 1680, 1530, 1420, 1370, 1110, 800, 750, 670, 590 cm⁻¹
MS m/z 369 (M⁺)

### Referential Example 17

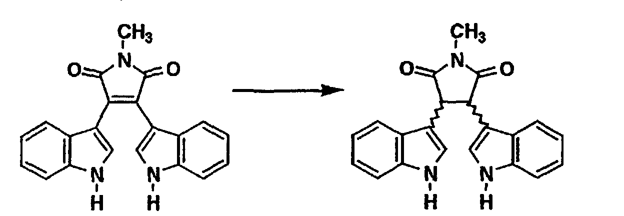

To a solution of Compound 14 (198 mg, 0.580 mmol) dissolved in DMF (10 mL) was added 10% palladium-carbon (40 mg), and the whole was stirred at room temperature for 1 day under hydrogen atmosphere. The palladium-carbon was removed by filtration, and the filtrate was concentrated under reduced pressure. The residue was purified by column chromatography over silica gel (ethyl acetate : n-hexane = 1 : 10 to 2 : 1) to obtain Compound 30 (191 mg, 96%) as a mixture of two isomers (A : B = 2.7 : 1.0) as pale yellow solids.
A: ¹H-NMR (CDCl₃) δ 3.28 (s, 3H), 4.77 (s, 2H), 6.6-7.4 (m, 10H), 7.68 (brs, 2H)
B: ¹H-NMR (CDCl₃) δ 3.26 (s, 3H), 4.41 (s, 2H), 6.6-7.4 (m, 10H), 8.15 (brs, 2H)
MS m/z 343 (M⁺)

### Referential Example 18

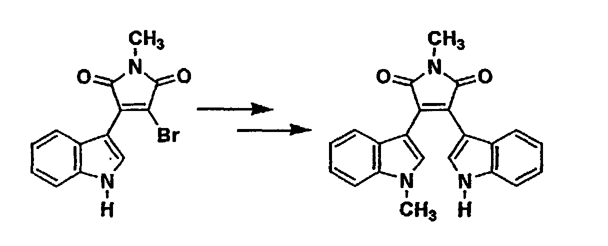

Potassium carbonate (140 mg, 0.98 mmol) and methyl iodide (0.06 mL, 0.98 mmol) were added under ice cooling to 2-bromo-3-(1H-indol-3-yl)-N-methylmaleimide (100 mg, 0.33 mmol) synthesized following to a known method (*Tetrahedron*, Vol. 44, p. 2887, 1988) and dissolved in DMF (5 mL), and the whole was stirred for 2 hours. The reaction mixture was warmed to room temperature, added with saturated aqueous sodium chloride solution, and extracted with ethyl acetate. The extract was dried over sodium sulfate, and then concentrated under reduced pressure. The residue was purified by column chromatography over silica gel (ethyl acetate : n-hexane = 1 : 1) to obtain 2-bromo-3-(1-methyl-1H-indol-3-yl)-N-methylmaleimide (99 mg, 95.1%) as red solids.
mp 155-158°C
¹H-NMR (CDCl₃) δ 3.17 (s, 3H), 3.89 (s, 3H), 7.16-7.41 (m, 4H), 8.05-8.11 (m, 1H)
IR (KBr) 1760, 1700, 1585, 1510, 1430, 1375, 1230, 1150, 1120, 980, 800, 730 cm⁻¹
MS m/z 318 (M⁺)

To a solution of indole (66 mg, 0.21 mmol) dissolved in toluene (1 mL) was added 0.95 M ethyhlmagnesium bromide (0.5 mL, 0.47 mmol) at 40°C, and the whole was stirred at 40°C for 45 minutes. Successively, a solution of 2-bromo-3-(1-methyl-1H-indol-3-yl)-N-methylmaleimide (66 mg, 0.21 mmol) dissolved in toluene (3 mL) was added thereto, followed by stirring under heating and refluxing for 2 hours. After 20% aqueous citric acid solution (1 mL) was added thereto under ice cooling and the whole was stirred, toluene was removed by concentration under reduced pressure and the resulting concentrate was extracted with ethyl acetate. The extract was dried over sodium sulfate and then concentrated under reduced pressure. The residue was purified by column chromatography over silica gel (ethyl acetate : n-hexane = 1 : 1) to obtain Compound 35 (71 mg, 96.8%) as red solids.
mp 168-171°C
¹H-NMR (DMSO-d₆) δ 3.05 (s, 3H), 3.35 (s, 3H), 6.65 (t, J=8,0Hz, 2H), 6.75 (d, J=8.0Hz, 1H), 6.85 (d, J=8.0Hz, 1H), 6.98 (t, J=8.0Hz, 1H), 7.03 (t, J=8.0Hz, 1H), 7.38 (d, J=8.1Hz, 1H), 7.42 (d, J=8.1Hz, 1H), 7.74 (d, J=2.8Hz, 1H), 7.82 (s, 1H), 11.66 (brs, 1H)
IR (KBr) 3450, 1700, 1540, 1440, 1380 cm⁻¹
MS m/z 355 (M⁺)

### Referential Example 19

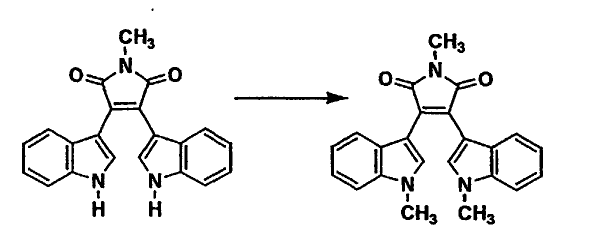

Compound 14 (50 mg, 0.15 mmol) synthesized following to a known method (*Tetrahedron*, Vol. 44, p. 2887, 1988) was dissolved in DMF (2 mL), and potassium carbonate (120 mg, 0.88 mmol) and methyl iodide (0.05 mL, 0.88 mmol) were added thereto under ice cooling. The whole was stirred for 19 hours. The reaction mixture was warmed to room temperature, added with saturated aqueous sodium chloride solution, and extracted with ethyl acetate. The extract was dried over sodium sulfate, and then concentrated under reduced pressure. The residue was purified by column chromatography over silica gel (ethyl acetate : n-hexane = 1 : 1) to obtain Compound 36 (54 mg, 99.4%) as red solids.
mp > 290°C
¹H-NMR (CDCl₃) δ 3.17 (s, 3H), 3.82 (s, 6H), 6.72 (t, J=8.1Hz, 2H), 6.91 (d, J=8.1Hz, 2H), 7.09 (t, J=8.1Hz, 2H), 7.27 (d, J=8.1Hz, 2H), 7.67 (s, 2H)
IR (KBr) 3390, 1695, 1530, 1440, 1365, 740 cm⁻¹
MS m/z 369 (M⁺)

### Referential Example 20

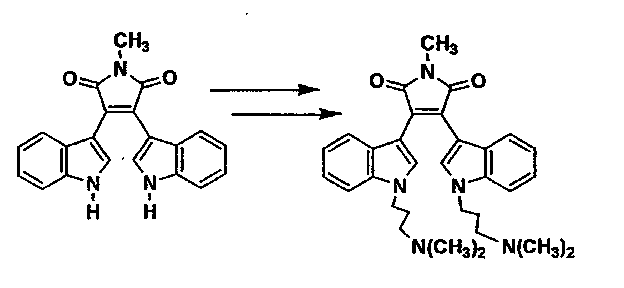

Sodium hydride (60 to 72%, oily, 50 mg) was washed with pentane and then suspended into DMF (0.3 mL). A DMF solution (1.2 mL) of Compound 14 (150 mg, 0.44 mmol) synthesized following to a known method (*Tetrahedron*, Vol. 44, p. 1887, 1988) was added thereto, and the whole was stirred at room temperature for 45 minutes. On the other hand, DMF (1.2 mL) was added at 0°C to a mixture of (3-chloropropyl)dimethylamine hydrochloride (70 mg, 0.44 mmol) and sodium hydride (60 to 75%, oily, 18 mg) washed with pentane, and the whole was stirred for 5 minutes and then warmed to room temperature with standing. The supernatant was added to the solution of sodium salt of Compound 14. The resulting mixture was stirred at 40°C for 1.5 hours, and then DMF was removed under reduced pressure. To the residue was added dichloromethane and saturated aqueous sodium chloride solution, and the organic layer was separated. The water layer was further extracted with dichloromethane. The resulting organic layers were combined and dried over sodium sulfate. The residue obtained by removing the solvent under reduced pressure was purified by column chromatography over silica gel (chloroform saturated with ammonia : methanol = 10 : 1) to obtain 2-(1-(3-dimethylaminopropyl)-1H-indol-3-yl)-3-(1H-indol-3-yl)-N-methylmaleimide (77 mg, 41%) as dark red solids.
mp 86-90°C
¹H-NMR (CDCl₃) δ 1.95 (tt, J=6.8, 6.8Hz, 2H), 2.21 (s, 6H), 2.23 (t, J=6.8Hz, 2H), 3.19 (s, 3H), 4.21 (t, J=6.8Hz, 2H), 6.60-6.82 (m, 2H), 6.95 (d, J=8.1 Hz, 1H), 7.00-7.12 (m, 3H), 7.26-7.35 (m, 2H), 7.67 (s, IH), 7.72 (d, J=2.7Hz, 1H), 8.60 (brs, 1H)
IR (KBr) 3395, 2950, 1700, 1535, 1440, 1390, 750 cm⁻¹
MS m/z 426 (M⁺)

Sodium hydride (60 to 72%, oily, 4.5 mg) was washed with pentane and then suspended into DMF (0.1 mL). A DMF solution (0.5 mL) of 2-(1-(3-dimethylaminopropyl)-1H-indol-3-yl)-3-(1H-indol-3-yl)-N-methylmaleimide (1.6 mg, 0.038 mmol) was added thereto, and the whole was stirred at room temperature for 45 minutes. On the other hand, DMF (0.5 mL) was added at 0°C to a mixture of (3-chloropropyl)dimethylamine hydrochloride (8.9 mg, 0.056 mmol) and sodium hydride (60 to 75%, oily, 2.2 mg) washed with pentane, and the whole was stirred for 5 minutes and then warmed to room temperature with standing. The supernatant was added to the above solution of the sodium salt. The resulting mixture was stirred at 40°C for 1.5 hours, and then DMF was removed under reduced pressure. To the residue were added dichloromethane and saturated aqueous sodium chloride solution, and the organic layer was separated. The water layer was further extracted with dichloromethane. The resulting organic layers were combined and dried over sodium sulfate. The residue obtained by removing the solvent under reduced pressure was purified by column chromatography over silica gel (chloroform saturated with ammonia : methanol = 10 : 1) to obtain compound 37 (4.4 mg, 23%) as red solids.
¹H-NMR (CDCl₃) δ 1.97 (tt, J=6.8, 6.8Hz, 4H), 2.20-2.32 (m, 16H), 3.18 (s, 3H), 4.19 (t, J=6.8Hz, 4H), 6.70 (t, J=8.1Hz, 2H), 6.97 (d, J=8.1Hz, 2H), 7.12 (t, J=8.1Hz, 2H), 7.31 (d, J=8.1Hz, 2H), 7.68 (s, 2H)
MS m/z 511 (M⁺)

### Referential Example 21

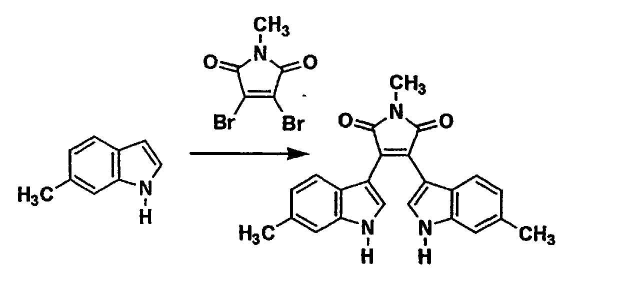

To a solution of 6-methylindole (250 mg, 2.13 mmol) dissolved in toluene (3 mL) was added 1.02 M ethylmagnesium bromide (2.1 mL, 2.13 mmol) at 40°C, and the whole was stirred at 40°C for 45 minutes. Successively, a solution of 2,3-dibromo-N-methylmaleimide (120 mg, 0.47 mmol) dissolved in toluene (7 mL) was added thereto, followed by stirring under heating and refluxing for 3 hours. After addition of 20% aqueous citric acid solution (3 mL) to the reaction mixture under ice cooling and successive stirring, toluene was removed by concentration under reduced pressure and the resulting concentrate was extracted with ethyl acetate. The extract was dried over sodium sulfate and then concentrated under reduced pressure. The residue was purified by column chromatography over silica gel (ethyl acetate : n-hexane = 1 : 1) to obtain Compound 38 (152 mg, 87.5%) as red solids.
mp 139-143°C
¹H-NMR (DMSO-d₆) δ 2.29 (s, 6H), 3.03 (s, 3H), 6.50 (d, J=8.2Hz, 2H), 6.73 (d, J=8.2Hz, 2H), 7.16 (s, 2H), 7.64 (d, J=2.7Hz, 2H), 11.49 (brs, 2H)
IR (KBr) 3390, 1695, 1535, 1445, 1390 cm⁻¹
MS m/z 369 (M⁺)

### Referential Example 22

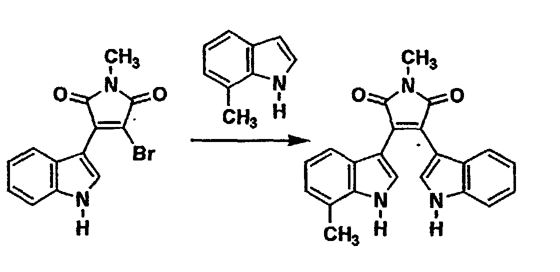

To a solution of 7-methylindole (190 mg, 1.63 mmol) dissolved in toluene (6 mL) was added 1.02 M ethylmagnesium bromide (1.6 mL, 1.63 mmol) at 40°C, and the whole was stirred at 40°C for 45 minutes. Successively, a solution of 2-bromo-3-(indol-3-yl)-N-methylmaleimide (300 mg, 0.74 mmol) synthesized following to a known method (*Tetrahedron*, Vol. 44, p. 2887, 1988) and dissolved in toluene (8 mL) was added thereto, followed by stirring under heating and refluxing for 3 hours. After addition of 20% aqueous citric acid solution (3 mL) to the reaction mixture under ice cooling and successive stirring, toluene was removed by concentration under reduced pressure and the resulting concentrate was extracted with ethyl acetate. The extract was dried over sodium sulfate and then concentrated under reduced pressure. The residue was purified by column chromatography over silica gel (ethyl acetate : n-hexane = 1 : 3 to 2 : 1) to obtain compound 39 (220 mg, 84.1%) as red solids.
mp 192-195°C
¹H-NMR (DMSO-d₆) δ 2.45 (s, 3H), 3.05 (s, 3H), 6.55 (t, J=7.6Hz, 1H), 6.65 (d, J=7.6Hz, 1H), 6.67 (t, J=7.6Hz, 1H), 6.78 (d, J=7.6Hz, 1H), 6.88 (d, J=7.6Hz, 1H), 6.99 (t, J=7.6Hz, 1H), 7.38 (d, J=7.6Hz, 1H), 7.72 (d, J=2,7Hz, 1H), 7.74 (d, J=2.4Hz, 1H), 11.65 (brs, 1H), 11.66 (brs, 1H)
IR (KBr) 3390, 1695, 1540, 1440, 1390, 750 cm⁻¹
MS m/z 355 (M⁺)

### Referential Example 23

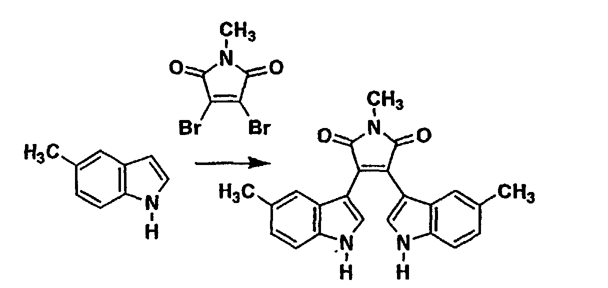

To a solution of 5-methylindole (320 mg, 2.75 mmol) dissolved in toluene (5 mL) was added 0.95 M ethylmagnesium bromide (2.8 mL, 2.75 mmol) at 40°C, and the whole was stirred at 40°C for 45 minutes. Successively, a solution of 2,3-dibromo-N-methylmaleimide (150 mg, 0.61 mmol) dissolved in toluene (3 mL) was added thereto, followed by stirring under heating and refluxing for 3 hours. After addition of 20% aqueous citric acid solution (3 mL) to the reaction mixture under ice cooling and successive stirring, toluene was removed by concentration under reduced pressure and the resulting concentrate was extracted with ethyl acetate. The extract was dried over sodium sulfate and then concentrated under reduced pressure. The residue was purified by column chromatography over silica gel (ethyl acetate : n-hexane = 1 : 1) to obtain Compound 40 (200 mg, 92.1%) as red solids.
mp 270-275°C
¹H-NMR (CDCl₃) δ 2.06 (s, 6H), 3.20 (s, 3H), 6.73 (s, 2H), 6.90 (d, J=8.3Hz, 2H), 7.20 (d, J=8.3Hz, 2H), 7.62 (d, J=2.8Hz, 2H), 8.42 (brs, 2H)
IR (KBr) 3310, 1690, 1525, 1440, 1390, 1160, 1105, 800 cm⁻¹
MS m/z 369 (M⁺)

### Referential Example 24

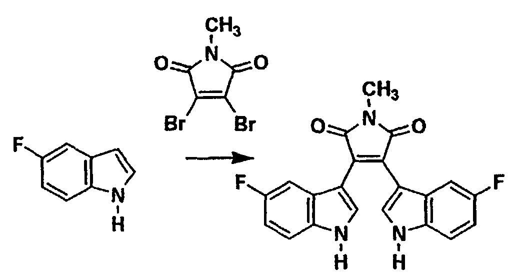

To a solution of 5-fluoroindole (240 mg, 1.77 mmol) dissolved in toluene (4 mL) was added 1.02 M ethylmagnesium bromide (1.7 mL, 1.77 mmol) at 40°C, and the whole was stirred at 40°C for 45 minutes. Successively, a solution of 2,3-dibromo-N-methylmaleimide (100 mg, 0.4 mmol) dissolved in toluene (6 mL) was added thereto, followed by stirring under heating and refluxing for 3 hours. After addition of 20% aqueous citric acid solution (2 mL) to the reaction mixture under ice cooling and successive stirring, toluene was removed by concentration under reduced pressure and the resulting concentrate was extracted with ethyl acetate. The extract was dried over sodium sulfate and then concentrated under reduced pressure. The residue was purified by column chromatography over silica gel (ethyl acetate : n-hexane = 1 : 1) to obtain Compound 41 (125 mg, 84.8%) as red solids.
mp > 290°C
¹H-NMR (DMSO-d₆) δ 3.03 (s, 3H), 6.38 (dd, J=9.0, 2.5Hz, 2H), 6.83 (dt, J=2.5, 9.0Hz, 2H), 7.39 (dd, J=9.0, 4.6Hz, 2H), 7.89 (m, 2H), 11.83 (brs, 2H)
IR (KBr) 3440, 3350, 1700, 1530, 1450, 1430 cm⁻¹
MS m/z 377 (M⁺)

### Referential Example 25

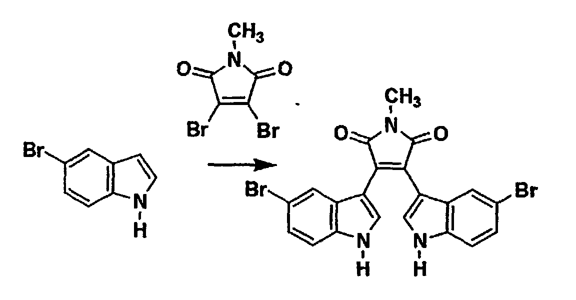

To a solution of 5-bromoindole (690 mg, 3.53 mmol) dissolved in toluene (6 mL) was added 0.95 M ethylmagnesium bromide (3.7 mL, 3.53 mmol) at 40°C, and the whole was stirred at 40°C for 45 minutes. Successively, a solution of 2,3-dibromo-N-methylmaleimide (200 mg, 0.78 mmol) dissolved in toluene (17 mL) was added thereto, followed by stirring under heating and refluxing for 3 hours. After addition of 20% aqueous citric acid solution (6 mL) to the reaction mixture under ice cooling and successive stirring, toluene was removed by concentration under reduced pressure and the resulting concentrate was extracted with ethyl acetate. The extract was dried over sodium sulfate and then concentrated under reduced pressure. The residue was purified by column chromatography over silica gel (ethyl acetate : n-hexane = 1 : 1) to obtain compound 42 (322 mg, 82.2%) as red solids.
mp > 290°C
¹H-NMR (DMSO-d₆) δ 3.05 (s, 3H), 6.86 (d, J=1.8Hz, 2H), 7.12 (dd, J=8.6, 1.8Hz, 2H), 7.38 (d, J=8.6Hz, 2H), 7.82 (d, J=2.7Hz, 2H), 11.89 (brs, 2H)
IR (KBr) 3380, 1695, 1640, 1540, 1460, 1440, 1395 cm⁻¹
MS m/z 499 (M⁺)

### Referential Example 26

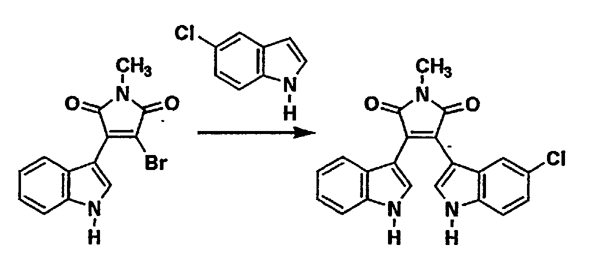

To a solution of 5-chloroindole (122 mg, 0.81 mmol) dissolved in toluene (3 mL) was added 0.96 M ethylmagnesium bromide (0.84 mL, 0.81 mmol) at 40°C, and the whole was stirred at 40°C for 45 minutes. Successively, a solution of 2-bromo-3-(1H-indol-3-yl)-N-methylmaleimide (70 mg, 0.23 mmol) synthesized following to a known method (*Tetrahedron*, Vol. 44, p. 2887, 1988) and dissolved in toluene (9 mL) was added thereto, followed by stirring under heating and refluxing for 2 hours. After addition of 20% aqueous citric acid solution (0.5 mL) to the reaction mixture under ice cooling and successive stirring, toluene was removed by concentration under reduced pressure and the resulting concentrate was extracted with ethyl acetate. The extract was dried over sodium sulfate and then concentrated under reduced pressure. The residue was purified by column chromatography over silica gel (ethyl acetate : n-hexane = 1 : 5 to 1 : 1) to obtain Compound 43 (89 mg, 100%) as red solids.
mp 114-117°C
¹H-NMR (CDCl₃) δ 3.20 (s, 3H), 6.60-7.40 (m, 7H), 7.70-7.90 (m, 2H), 8.50 (brs, 1H), 8.60 (brs, 1H)
IR (KBr) 3350, 1680, 1520, 1440, 1370, 735 cm⁻¹
MS m/z 375 (M⁺)

### Referential Example 27

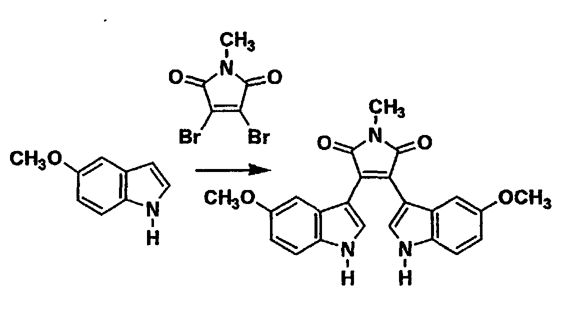

To a solution of 5-methoxyindole (520 mg, 3.53 mmol) dissolved in toluene (8 mL) was added 0.95 M ethylmagnesiumn bromide (3.7 mL, 3.53 mmol) at 40°C, and the whole was stirred at 40°C for 45 minutes. Successively, a solution of 2,3-dibromo-N-methylmaleimide (200 mg, 0.73 mmol) dissolved in toluene (12 mL) was added thereto, followed by stirring under heating and refluxing for 3 hours. After addition of 20% aqueous citric acid solution (2 mL) to the reaction mixture under ice cooling and successive stirring, toluene was removed by concentration under reduced pressure and the resulting concentrate was extracted with ethyl acetate. The extract was dried over sodium sulfate and then concentrated under reduced pressure. The residue was purified by column chromatography over silica gel (ethyl acetate : n-hexane = 1 : 1) to obtain Compound 44 (136 mg, 86.4%) as red solids.
mp > 290°C
¹H-NMR (DMSO-d₆) δ 3.03 (s, 3H), 3.33 (s, 6H), 6.21 (d, J=2.3Hz, 2H), 6.55 (dd, J=8.7, 2.3Hz, 2H), 7.23 (d, J=8.7Hz, 2H), 7.77 (d, J=2.8Hz, 2H), 11.55 (brd, J=2.8Hz, 2H)
IR (KBr) 3325, 1690, 1530, 1460, 1440, 1220, 1165, 805 cm⁻¹
MS m/z 401 (M⁺)

### Referential Example 28

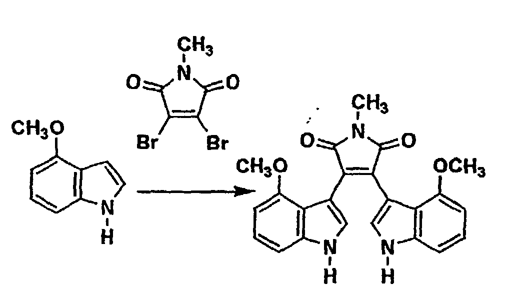

To a solution of 4-methoxyindole (100 mg, 0.68 mmol) dissolved in toluene (1 mL) was added 0.95 M ethylmagnesium bromide (0.72 mL, 0.68 mmol) at 40°C, and the whole was stirred at 40°C for 45 minutes. Successively, a solution of 2,3-dibromo-N-methylmaleimide (40 mg, 0.15 mmol) dissolved in toluene (3 mL) was added thereto, followed by stirring under heating and refluxing for 3 hours. After addition of 20% aqueous citric acid solution (2 mL) to the reaction mixture under ice cooling and successive stirring, toluene was removed by concentration under reduced pressure and the resulting concentrate was extracted with ethyl acetate. The extract was dried over sodium sulfate and then concentrated under reduced pressure. The residue was purified by column chromatography over silica gel (ethyl acetate : n-hexane = 1 : 2 to 2 : 1) to obtain Compound 45 (57 mg, 90.5%) as red solids.
mp 182-186°C
¹H-NMR (DMSO-d₆) δ 3.04 (s 3H), 3.45 (s, 6H), 6.37 (d, J=7.9Hz, 2H), 6.94 (d, J=7.9Hz, 2H), 6.98 (t, J=7.9Hz, 2H), 7.25 (brs, 2H), 11.31 (brs, 2H)
IR (KBr) 3390, 1700, 1440, 735 cm⁻¹
MS m/z 401 (M⁺)

### Referential Example 29

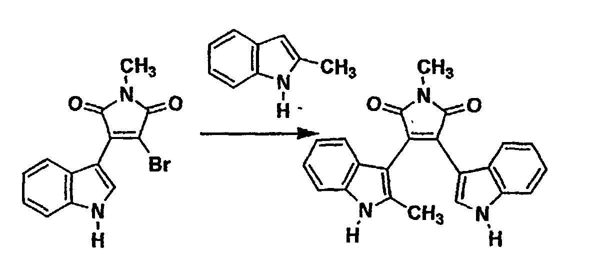

To a solution of 2-methylindole (190 mg, 1.63 mmol) dissolved in toluene (6 mL) was added 1.02 M ethylmagnesium bromide (1.6 mL, 1.63 mmol) at 40°C, and the whole was stirred at 40°C for 45 minutes. Successively, thereto was added a solution of 2-bromo-3-(1H-indol-3-yl)-N-methylmaleimide (300 mg, 0.74 mmol) synthesized following to a known method (*Tetrahedron*, Vol. 44, p. 2887, 1988) and dissolved in toluene (8 mL), followed by stirring under heating and refluxing for 3 hours. After addition of 20% aqueous citric acid solution (3 mL) to the reaction mixture under ice cooling and successive stirring, toluene was removed by concentration under reduced pressure and the resulting concentrate was extracted with ethyl acetate. The extract was dried over sodium sulfate and then concentrated under reduced pressure. The residue was purified by column chromatography over silica gel (ethyl acetate : n-hexane = 1 : 3 to 2 : 1) to obtain Compound 46 (240 mg, 91.0%) as red solids.
mp 183-186°C
¹H-NMR (DMSO-d₆) δ 1.91 (s, 3H), 3.10 (s, 3H), 6.47 (d, J=7.6Hz, 1H), 6.59 (t, J=7.6Hz, 1H), 6.92 (t, J=7.6Hz, 1H), 7.03 (t, J=7.6Hz, 1H), 7.07 (t, J=7.6Hz, 1H), 7.32 (d, J=7.6Hz, 1H), 7.35 (d, J=7.6Hz, 1H), 7.42 (d, J=7.6Hz, 1H), 7.99 (m, 1H), 11.32 (brs, 1H), 11.80 (brs, 1H)
IR (KBr) 3390, 1695, 1460, 1440, 1392, 740 cm⁻¹
MS m/z 355 (M⁺)

### Referential Example 30

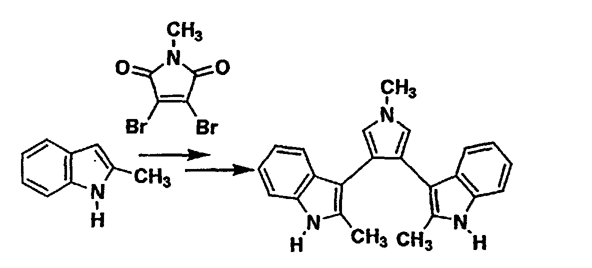

To a solution of 2-methylindole (1.0 g, 8.82 mmol) dissolved in THF (10 mL) was added 0.95 M ethylmagnesium bromide (9.3 mL, 8.82 mmol) at 40°C, and the whole was stirred at 40°C for 45 minutes. Successively, a solution of 2,3-dibromo-N-methylmaleimide (500 mg, 1.96 mmol) dissolved in THF (4 mL) was added thereto, followed by stirring under heating and refluxing for 3 hours. After addition of 20% aqueous citric acid solution (9 mL) to the reaction mixture under ice cooling and successive stirring, THF was removed by concentration under reduced pressure and the resulting concentrate was extracted with ethyl acetate. The extract was dried over sodium sulfate and then concentrated under reduced pressure. The residue was purified by column chromatography over silica gel (ethyl acetate : n-hexane = 1 : 2) to obtain Compound 47 (452 mg, 62.4%) as red solids.
mp > 290°C
¹H-NMR (CDCl₃) δ 2.06 (s, 6H), 3.22 (s, 3H), 6.92 (t, J=7.6Hz, 2H), 7.08 (t, J=7.6Hz, 2H), 7.15-7.24 (m, 4H), 8.04 (brs, 2H)
IR (KBr) 3380, 3310, 1705, 1460, 1440, 1380 cm⁻¹
MS m/z 369 (M⁺)

### Referential Example 31

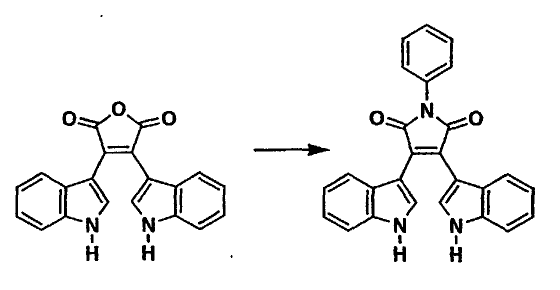

To a solution of Compound 11 (100 mg, 0.3 mmol) synthesized following to a known method (*Tetrahedron*, Vol. 44, p. 2887, 1988) and dissolved in DMF (10 mL) and water (10 mL) was added aniline (0.12 mL, 1.2 mmol), and the whole was stirred at 100°C for 2 hours. The reaction mixture was concentrated under reduced pressure to remove DMF, and the concentrate was extracted with ethyl acetate. The extract was washed with water, dried over sodium sulfate, and then concentrated under reduced pressure. The residue was purified by column chromatography over silica gel (ethyl acetate : n-hexane = 2 : 1) to obtain Compound 48 (116 mg, 94.4%) as red solids.
mp > 290°C
¹H-NMR (CDCl₃) δ 6.77 (t, J=7.1Hz, 2H), 7.04 (d, J=7.1Hz, 2H), 7.10 (t, J=7.1Hz, 2H), 7.36 (d, J=7.1Hz, 2H), 7.35-7.45 (m, 1H), 7.48-7.55 (m, 4H), 7.85 (d, J=2.8Hz, 2H), 8.54 (brs, 2H)
IR (KBr) 3365, 1700, 1525, 1430, 1390, 1245, 1120, 740 cm⁻¹
MS m/z 403 (M⁺)

### Referential Example 32

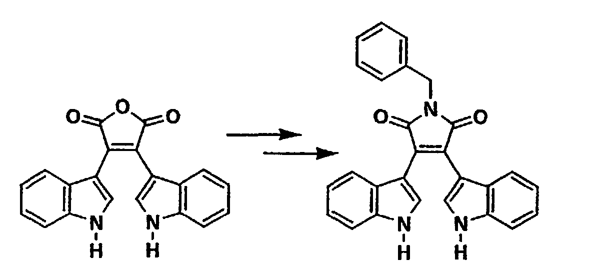

To a solution of Compound 11 (100 mg, 0.3 mmol) synthesized following to a known method (*Tetrahedron*, Vol. 44, p. 2887, 1986) and dissolved in DMF (10 mL) and water (10 mL) was added benzylamine (0.13 mL, 1.2 mmol), and the whole was stirred at 100°C for 2 hours. The reaction mixture was concentrated under reduced pressure to remove DMF, and the concentrate was extracted with ethyl acetate. The extract was washed with water, dried over sodium sulfate, and then concentrated under reduced pressure. The residue was purified by column chromatography over silica gel (ethyl acetate n-hexane = 2 : 1) to obtain Compound 49 (110 mg, 86.6%) as red solids.
mp > 290°C
¹H-NMR (CDCl₃) δ 4.86 (s, 2H), 6.75 (t, J=8.0Hz, 2H), 6.97 (d, J=8.0Hz, 2H), 7.06 (t, J=8.0Hz, 2H), 7.28-7.38 (m, 5H), 7.50 (d, J=8.0Hz, 2H), 7.7 (d, J=2.8Hz, 2H), 8.49 (brs, 2H)
IR (KBr) 3400, 1695, 1530, 1430, 1405, 750 cm⁻¹
MS m/z 417 (M⁺)

### Referential Example 33

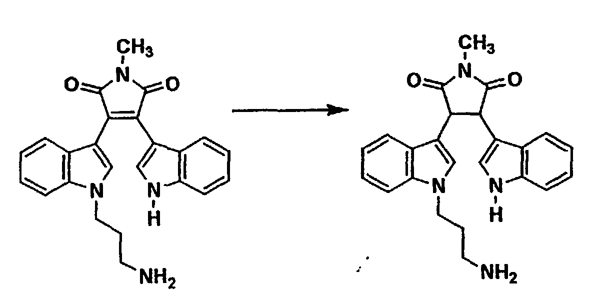

A small amount of 10% palladium-carbon was added to a solution of Compound 19 (48 mg, 0.12 mmol) dissolved in DMF (1 mL), and the whole was stirred at room temperature for 2 days under hydrogen atmosphere. The palladium-carbon was removed by filtration, and the filtrate was concentrated under reduced pressure. The residue was purified by column chromatography over silica gel (chloroform saturated with ammonia : methanol = 10 : 1) to obtain Compound 33 (32 mg, 66.7%) as pale red solids.
¹H-NMR (CDCl₃) δ 1.42 (brs, 2H), 1.90 (tt, J=6.8, 6.8Hz, 2H), 2.67 (t, J=6.8Hz, 2H), 3.26 (s, 3H), 4.12 (t, J=6.8 Hz, 2H), 4.42 (s, 2H), 6.82-7.45 (m, 10H), 8.52 (brs, 1H)

### Referential Example 34

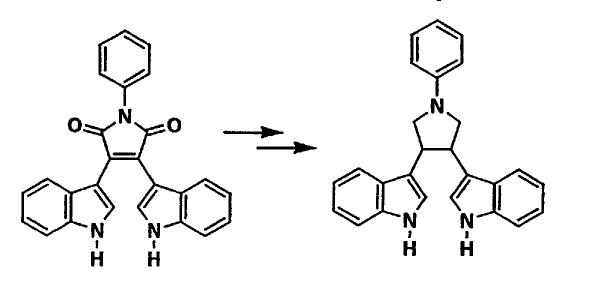

A small amount of 10% palladium-carbon was added to a solution of Compound 48 (55 mg, 0.14 mmol) dissolved in DMF (2 mL), and the whole was stirred at room temperature for 1 day under hydrogen atmosphere. The palladium-carbon was removed by filtration, and the filtrate was concentrated under reduced pressure. The residue was purified by column chromatography over silica gel (ethyl acetate : n-hexane = 2 : 1) to obtain 3,4-bis(1H-indol-3-yl)-1-phenyl-2,5-dioxopyrrolidine (36 mg, 56.1%) as pale red solids.
mp 260-263°C
¹H-NMR (CDCl₃) δ 4.92 (s, 2H), 6.61-6.70 (m, 2H), 6.87-7.09 (m, 6H), 7.30-7.60 (m, 7H), 7.64 (brs, 2H)
IR (KBr) 3440, 3400, 1700, 1380, 1180, 750 cm⁻¹
MS m/z 405 (M⁺)

Into THF (1 mL) was dissolved 3,4-bis(1H-indol-3-yl)-1-phenyl-2,5-dioxopyrrolidine (30 mg, 0.07 mmol), and thereto was added 0.94 M diisobutylaluminum hydride (0.3 mL, 0.29 mmol) dropwise slowly under ice cooling. After stirring at room temperature for 4 hours, the reaction mixture was added with saturated aqueous ammonium chloride solution and then stirred for further 30 minutes. Insoluble matter was removed by filtration through celite, and the filtrate was concentrated under reduced pressure. The residue was purified by column chromatography over silica gel (ethyl acetate : n-hexane = 1 : 1) to obtain Compound 34 (4.4 mg, 15.8%) as colorless solids.
mp 97-99°C
¹H-NMR (CDCl₃) δ 3.74 (dd, J=5.4, 9.0Hz, 2H), 3.92 (dd, J=6.5, 9.0Hz, 2H), 4.13-4.25 (m, 2H), 6.46 (d, J=2.4Hz, 2H), 6.96-6.78 (m, 3H), 6.91 (t, J=8.0Hz, 2H), 7.07 (t, J=8.0Hz, 2H), 7.20 (d, J=8.0Hz, 2H), 7.26-7.37 (m, 4H), 7.64 (brs, 2H)
IR (KBr) 3410, 1600, 1510, 1480, 1460, 1370, 745 cm⁻¹
MS m/z 377 (M⁺)

From the above results, it was found that the compounds according to the present invention inhibit cell death of various cells induced-by various stimuli. Also, it was found that cell death inhibitors could be conveniently detected by the assay method wherein cell death induced by various apoptotic stimuli was determined by means of a microscope or a coloring test.

### Industrial Applicability

Since the indole derivatives according to the present invention inhibit cell death caused by various cell death-inducing stimuli, they are considered to be useful for prevention or treatment of all the diseases wherein cell death participates in outlook and exacerbation thereof. Accordingly, the inhibitors have uses as remedies for neurodegenerative diseases such as Alzheimer's disease, spinal muscular atrophy, amyotrophic lateral screrosis, Parkinson's disease, Huntington's disease, pigmentary degeneration of the retina, glaucoma, cerebellar degeneration and neonatal jaundice; myasthenia gravis; brain ischemia from apoplexy and the like, and successive delayed neuronal death (DND), ischemic heart disease due to myocardial infarction (myocardial ischemia and disorder after reperfusion); viral myocarditis; autoimmune myocarditis (congestive cardiomyopathy and chronic myocarditis); myocardial disorders or death due to hypertrophic heart and heart failure; arrythmogenic right ventricular cardiomyopathy; alcoholic hepatitis and viral hepatitis; renal diseases such as glomerulonephritis, hemolytic uremic syndrome and the like; acquired immunodeficiency syndrome (AIDS); inflammatory skin disorders such as toxic epidermal necrolysis (TEN) and multiform exudative erythema; alopecia; graft versus host disease (GVH); radiation disorders; disorders due to toxic agents including side effects due to anti-cancer drugs, anti-viral drugs and the like; sepsis; osteomyelo-dysplasia such as aplastic anemia and the like; insulin-dependent diabetes; prion diseases such as Creutzfeldt-Jakob's disease; and functional deficiency of transplanted organs and the like, or uses as drugs for stopping or inhibiting progress and exacerbation of the diseases; and also uses as preservatives for organs, tissues and cells. In addition, the assay system according to the present invention wherein primary cultured cells are used is considered to be useful for searching cell death inhibitors.

## Claims

1. A cell death inhibitor comprising, as an active ingredient, an indole derivative represented by the following formula (I): 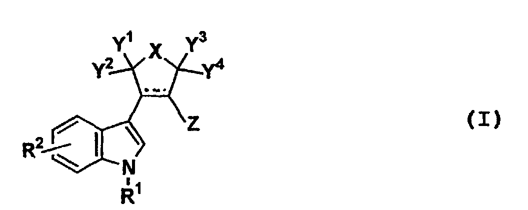 wherein X represents an oxygen atom or N-R⁵; Z represents a hologen atom or 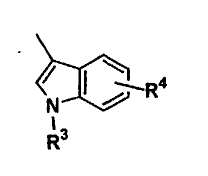 R¹ and R³ each independently represents a hydrogen atom, an alkyl group which may possess substituent(s), an alkenyl group which may possess substituent(s), an alkynyl group which may possess substituent(s), an aryl group which may possess substituent(s), an acyl group which may possess substituent(s), an alkoxy- or aryloxycarbonyl group which may possess substituent(s), an alkyl- or arylthiocarbonyl group which may possess substituent(s), an aminocarbonyl group which may possess substituent(s), an alkyl- or arylsulfonyl group which may possess substituent(s), an alkoxyl group or an aryloxy group which may possess substituent(s), or a hydroxyl group; R² and R⁴ each represents substituent(s) on an indole ring, in which number -and position (2-, 4-, 5-, 6-, or 7-position as position number of the indole ring) of the substituent(s) and kinds of the substituent(s) may be the same or different, and represents a hydrogen atom, an alkyl group which may possess substituent(s), an alkenyl group which may possess substituent(s), an alkynyl group which may possess substituent(s), an aryl group which may possess substituent(s), an acyl group which may possess substituent(s), an alkoxy- or aryloxycarbonyl group which may possess substituent(s), an alkyl- or arylthiocarbonyl group which may possess substituent(s), an aminocarbonyl group which may possess substituent(s), an alkyl- or arylsulfonyl group which may possess substituent(s), an alkoxyl group or an aryloxy group which may possess substituent(s), an alkyl-or arylthio group which may possess substituent(s), a hydroxyl group, a carboxyl group, a cyano group, a nitro group, an amino group which may possess substituent(s), or a halogen atom; R⁵ represents an alkyl group which may possess substituent(s), an alkenyl group which may possess substituent(s), an alkynyl group which may possess substituent(s), an aryl group which may possess substituent(s), an alkoxyl group or an aryloxy group which may possess substituent(s), an amino group which may possess substituent(s), a hydroxyl group, or a hydrogen atom; Y¹ and Y², and Y³ and Y⁴ each independently represent two hydrogen atoms or a hydrogen atom and a hydroxyl group, or are combined to form a carbonyl group; and R¹ and R², R¹ and R³, R³ and R⁴, or R² and R⁴ may be combined to form a hydrocarbon chain or a hydrocarbon chain containing hetero atom(s) which may possess substituent(s); and in the formula, the bond accompanying a dotted line represents a double bond or a single bond, or a pharmaceutically acceptable salt thereof.

2. A drug for treating or preVenting progress of symptoms, through inhibiting death of neurons, of neurodegenerative diseases, comprising a derivative represented by the above formula (I) or a pharmaceutically acceptable salt thereof as an active ingredient.

3. A drug for treating or preventing progress of symptoms, through inhibiting death of neurons, of neonatal jaundice, comprising a derivative represented by the above formula (I) or a pharmaceutically acceptable salt thereof as an active ingredient.

4. A drug for treating or preventing progress of symptoms, through inhibiting cell death, of myasthenia gravis, comprising a derivative represented by the above formula (I) or a pharmaceutically acceptable salt thereof as an active ingredient.

5. A drug for treating or preventing progress of symptoms, through inhibiting death of neurons, of brain ischemia and delayed neuronal death (DND), comprising a derivative represented by the above formula (I) or a pharmaceutically acceptable salt thereof as an active ingredient.

6. A drug for treating or preventing progress of symptoms, through inhibiting death of myocardial cells, of ischemic heart disease, viral myocarditis, autoimmune myocarditis, myocardial disorders or cell death due to hypertrophic heart and heart failure, or arrythmogenic right ventricular cardiomyopathy, comprising a derivative represented by the above formula (I) or a pharmaceutically acceptable salt thereof as an active ingredient.

7. A drug for treating or preventing progress of symptoms, through inhibiting death of hepatic cells, of alcoholic hepatitis or viral hepatitis, comprising a derivative represented by the above formula (I) or a pharmaceutically acceptable salt thereof as an active ingredient.

8. A drug for treating or preventing progress of symptoms, through inhibiting death of renal cells, of renal diseases, comprising a derivative represented by the above formula (I) or a pharmaceutically acceptable salt thereof as an active ingredient.

9. A drug for treating or preventing progress of symptoms, through inhibiting excessive death of T-cells, of acquired immunodeficiency syndrome (AIDS), comprising a derivative represented by the above formula (I) or a pharmaceutically acceptable salt thereof as an active ingredient.

10. A drug for treating or preventing progress of symptoms, through inhibiting cell death, of inflammatory skin disorders, alopecia, or graft versus host disease (GVH), comprising a derivative represented by the above formula (I) or a pharmaceutically acceptable salt thereof as an active ingredient.

11. A drug for treating or preventing disorders or side effects, through inhibiting cell death, of radiation disorders, or disorders or side effects due to toxic agents, comprising a derivative represented by the above formula (I) or a pharmaceutically acceptable salt thereof as an active ingredient.

12. A drug for treating or preventing progress of symptoms, through inhibiting cell death, of sepsis, comprising a derivative represented by the above formula (I) or a pharmaceutically acceptable salt thereof as an active ingredient.

13. A drug for treating or preventing progress of symptoms, through inhibiting death of cells derived from bone marrow, of osteomyelo-dysplasia, comprising a derivative represented by the above formula (I) of a pharmaceutically acceptable salt thereof as an active ingredient.

14. A drug for treating or preventing progress of symptoms, through inhibiting cell death, of insulin dependent diabetes, comprising a derivative represented by the above formula (I) or a pharmaceutically acceptable salt thereof as an active ingredient.

15. A drug for treating or preventing progress of symptoms, through inhibiting death of neurons, of prion diseases, comprising a derivative represented by the above formula (I) or a pharmaceutically acceptable salt thereof as an active ingredient.

16. A drug for treating or preventing functional deficiency of transplanted organs, tissues or cells at transplantation of organs, tissues or cells, comprising a derivative represented by the above formula (I) or a pharmaceutically acceptable salt thereof as an active ingredient.

17. A preservative for organs, tissues or cells, comprising a derivative represented by the above formula (I) or a pharmaceutically acceptable salt thereof as an active ingredient.

18. An assay method for cell death inhibiting substances, comprising applying a cell death-inducing stimulus to primary cultured cells in the presence of a test compound or adding a test compound just after applying a cell death-inducing stimulus, followed by evaluating a ratio of cell death.

19. A medicament comprising, as an active ingredient, a 2-halo-3-indolylmaleimide derivative represented by the following formula (II): 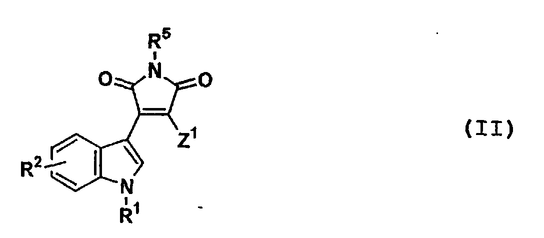 wherein Z¹ represents a halogen atom; and R¹, R² and R⁵ have the same meanings as described above, or a pharmaceutically acceptable salt thereof.
